(19) Europäisches Patentamt
European Patent Office
Office européen des brevets

(11) EP 3 632 918 B1

(12) **EUROPEAN PATENT SPECIFICATION**

(45) Date of publication and mention of the grant of the patent:
**20.12.2023 Bulletin 2023/51**

(21) Application number: **17911514.2**

(22) Date of filing: **06.06.2017**

(51) International Patent Classification (IPC):
***C07D 519/00*** *(2006.01)* ***C07D 471/08*** *(2006.01)*
***C07D 487/04*** *(2006.01)* ***A61K 31/551*** *(2006.01)*
***A61K 31/501*** *(2006.01)* ***A61K 31/4439*** *(2006.01)*
***A61P 25/28*** *(2006.01)* ***A61P 25/18*** *(2006.01)*
***A61P 25/14*** *(2006.01)* ***A61K 51/04*** *(2006.01)*
***A61K 101/02*** *(2006.01)*

(52) Cooperative Patent Classification (CPC):
**A61K 51/0468; A61K 51/0455; A61K 51/0459; A61P 25/00; C07B 59/002; C07D 471/08; C07D 487/04; C07D 519/00**

(86) International application number:
**PCT/CN2017/087339**

(87) International publication number:
**WO 2018/218696 (06.12.2018 Gazette 2018/49)**

(54) **LIGAND COMPOUND OF 7 NICOTINIC ACETYLCHOLINE RECEPTOR AND APPLICATION THEREOF**

LIGANDENVERBINDUNG VON 7-NIKOTINISCHEM ACETYLCHOLINREZEPTOR UND ANWENDUNG DAVON

COMPOSÉ LIGAND D'UN RÉCEPTEUR NICOTINIQUE DE L'ACÉTYLCHOLINE 7 ET SON APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2017 CN 201710395513**

(43) Date of publication of application:
**08.04.2020 Bulletin 2020/15**

(73) Proprietor: **Beijing Normal University**
**Beijing 100875 (CN)**

(72) Inventors:
- **ZHANG, Huabei**
  **Beijing 100875 (CN)**
- **WANG, Huan**
  **Beijing 100875 (CN)**
- **FANG, Yu**
  **Beijing 100875 (CN)**
- **LIU, Jianping**
  **Beijing 100875 (CN)**
- **WANG, Shuxia**
  **Beijing 100875 (CN)**

(74) Representative: **Office Freylinger**
**P.O. Box 48**
**8001 Strassen (LU)**

(56) References cited:
**EP-A2- 1 219 622**
**WO-A1-2006/051413**
**WO-A1-2007/017750**
**WO-A2-2013/057687**
**CN-A- 101 189 233**
**CN-A- 101 370 809**
**TW-A- 200 628 476**
**TW-A- 200 631 956**
**US-A1- 2005 101 602**
**US-A1- 2005 171 079**
**US-A1- 2005 234 031**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- C. J. O'DONNEL ET AL.: "Discovery of 4-(5-methyloxazolo[4,5-b]pyridin-2-yl)-1,4 -diazabicyclo[3.2.2]nonane (CP-810,123), a novel alpha 7 nicotinic acetylcholine receptor agonist for the treatment of cognitive disorders in schizophrenia: synthesis, SAR development, and in vivo efficacy in cognition models", J. MED. CHEM., vol. 53, 31 December 2009 (2009-12-31), pages 1222-1237, XP002797589,
- BEINAT ET AL.: "The recent development of alpha7 nicotinic acetylcholine receptor (nAChR) ligands as therapeutic candidates for the treatment of central nervous system (CNS) diseases", CURRENT PHARMACEUTICAL DESIGN, vol. 22, 2016, pages 2134-2151, XP002797590,
- Schrimpf, M.R. et al.: "SAR of 7 nicotinic receptor agonists derived from tilorone: Exploration of a novel nicotinic pharmacophore", Bioorganic & Medicinal Chemistry Letters, vol. 22, no. 4, 15 February 2012 (2012-02-15), pages 1633-1638, XP028398230, DOI: 10.1016/j.bmcl.2011.12.126
- Bunnelle, W.H.: "Octahydropyrrolo[3,4-c]pyrrole: A Diamine Scaffold for Construction of Either 4 2 or 7-Selective Nicotinic Acetylcholine Receptor (nAChR) Ligands. Substitutions that Switch Subtype Selectivity", Journal of Medicinal Chemistry, vol. 52, no. 14, 24 June 2009 (2009-06-24) , pages 4126-4141, XP002732635, DOI: 10.1021/jm900249k
- "Migration of Carbonyl Group in Subergorgic Acid Molecule"; "2" In: "Acta Scientiarum Naturalium Universitatis Sunyatseni", 1996 vol. 35, pages 9-12,

## Description

The present disclosure claims the priority of the Chinese patent application with the filing no. 201710395513.0, filed on May 27, 2017 with the Chinese Patent Office, entitled "Ligand Compound of α7 Nicotinic Acetylcholine Receptor and Application thereof".

## Technical Field

The present disclosure relates to the field of medical technologies, and particularly to a ligand compound of α7 nicotinic acetylcholine receptor and application thereof.

## Background Art

Nicotinic acetylcholine receptor (nAChR) is a class of gated-transmitter ion channels that are ubiquitous in the central nervous system (CNS) and the peripheral nervous system (PNS), and are associated with a variety of physiological functions. There are different subtypes of nAChR, which are generally composed of an α subunit (e.g., α2-α10) and a β subunit (β2-β4). Among them, α7 nicotinic acetylcholine receptor (α7 nAChR) is a homopentamer composed of 5 completely identical α subunits, and is mainly present in important regions related to memory, learning, etc., such as hippocampus, thalamus and cerebral cortex. Recent clinical research finds that the reduction of α7 nAChR protein density is found in brains of patients with neurodegenerative diseases such as Alzheimer's disease and Parkinson's disease, and the research on gene knockout, subtype selective ligands and the like shows that: the targeting α7 nAChR ligand can improve the cognitive ability and auditory gating defect, for example, high-selectivity α7 nAChR agonists such as PNU-282987, PHA-543613 and A-582941, improve the cognitive functions of models such as sensory-gating defect, short-term working memory, memory consolidation and the like. Therefore, synthesis of agonists and radioligands for the α7 nicotinic acetylcholine receptor is also receiving increasing attention.

## Summary

An object of embodiments of the present disclosure is to provide a ligand compound of α7 nicotinic acetylcholine receptor so as to obtain a radioactive or non-radioactive ligand molecule having high affinity with α7 nicotinic acetylcholine receptor. A specific technical solution was as follows:
The present disclosure provides a compound, having any one of the following formula:

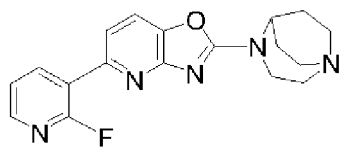
,
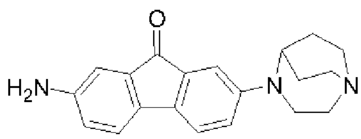
,
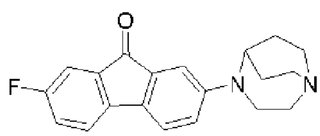
,
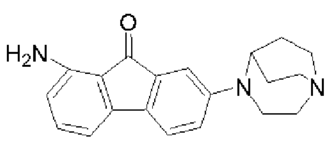
,
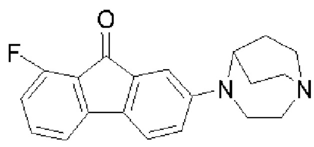
, or
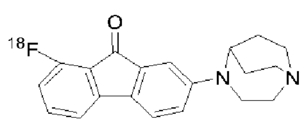
.

In some embodiments, the compound is used for preventing or treating a disease associated with α7 nicotinic acetylcholine receptor, and the disease associated with α7 nicotinic acetylcholine receptor is cognitive disorder being at least one selected from the group consisting of: presenile dementia, prese nile Alzheimer's disease, senile dementia, dementia of the Alzheimer's type, Lewy body corpuscle dementia, micro-infarct dementia, AIDS-related dementia, HIV dementia, dementia associated with Lewy body, dementia associated with Down's syndrome, Pick's disease, mild cognitive dysfunction, age-related memory disorder, recent short-term memory disorder, age-related cognitive disorder, drug-related cognitive disorder, cognitive disorder associated with immunodeficiency syndrome, cognitive dysfunction associated with vascular diseases, schizophrenia, attention deficit disorder, attention deficit hyperactivity disorder and learning deficit disorder.
In some embodiments, the compound is for use in therapy.
The present disclosure further provides a pharmaceutical composition containing a therapeutically effective amount of the abovementioned compound and a pharmaceutically acceptable carrier.

In some embodiments, the pharmaceutical composition is for use in therapy.

The present disclosure further provides a pharmaceutical composition for use in preventing or treating cognitive disorders, containing a therapeutically effective amount of the abovementioned compound and a pharmaceutically acceptable carrier, wherein the cognitive disorders being at least one selected from the group consisting of: presenile dementia, presenile Alzheimer's disease, senile dementia, dementia of the Alzheimer's type, Lewy body corpuscle dementia, micro-infarct dementia, AIDS-related dementia, HIV dementia, dementia associated with Lewy body, dementia associated with Down's syndrome, Pick's disease, mild cognitive dysfunction, age-related memory disorder, recent short-term memory disorder, age-related cognitive disorder, drug-related cognitive disorder, cognitive disorder associated with immunodeficiency syndrome, cognitive dysfunction associated with vascular diseases, schizophrenia, attention deficit disorder, attention deficit hyperactivity disorder and learning deficit disorder.

The present disclosure further provides use of a compound as a PET imaging agent, wherein the compound has a structural formula of

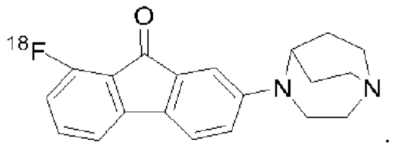

## Brief Description of Drawings

In order to more clearly illustrate technical solutions of examples of the present disclosure and the prior art, accompanying drawings that need to be used in the examples and the prior art are briefly introduced below, and obviously, the accompanying drawings in the following description are merely for some examples of the present disclosure, but a person ordinarily skilled in the art further could obtain other accompanying drawings according to these accompanying drawings without using creative efforts.

FIG. 1 is an HPLC chromatogram of [$^{18}$F] II-15 and II-15.

FIG. 2 shows a specific binding curve of binding between [$^{125}$I] $\alpha$-bgt and $\alpha$7 nAChRs membrane protein.

FIG. 3 shows a Hill straight line of binding between [$^{125}$I] $\alpha$-bgt and receptor membrane protein.

FIG. 4 is a Scatchard straight line.

FIG. 5 is a graph of probit Y and LogD(X).

FIG. 6 is an HPLC chart for stability analysis of radioligand [$^{18}$F] II-15 in fetal bovine serum and normal saline.

FIG. 7 shows results of selective experiment of [$^{18}$F] II-15 in mouse brains.

FIG. 8 shows PET imaging views of female CD-1 rats.

## Detailed Description of Embodiments

In order to make objects, technical solutions, and advantages of the present disclosure more clear and understandable, the present disclosure is further illustrated in detail below with reference to accompanying drawings and examples. Obviously, the examples described are merely some but not all examples of the present disclosure. Based on the examples in the present disclosure, all of other examples obtained by a person ordinarily skilled in the art, without using creative efforts, should belong to the scope of protection of the present disclosure.

Example 1: Synthesis of

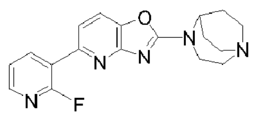

(called as compound I-9 for short)

A synthetic route of the compound I-9 was as follows:

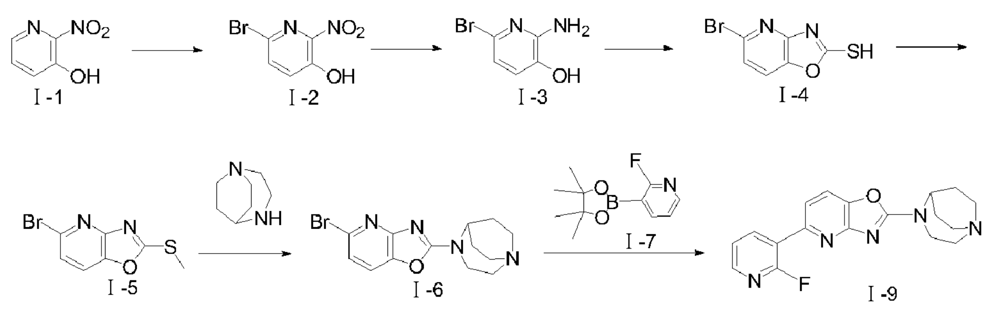


(1) Synthesis of compound I-2

To a clean and dry reaction flask a compound I-1 (5 g, 35.7 mmol) was added, and dissolved with 50 mL of methanol. After three times of replacement with nitrogen, a methanol solution (15 mL) of sodium methoxide (2.89 g, 53.5 mmol) was dropwise added to the reaction system, followed by stirring at room temperature for 30 min; then the reaction system was cooled to 0 °C, bromine (6.3 g, 39.4 mmol) was dropwise added, and the reaction system was controlled at the temperature of 0-5 °C and reacted for 4 h; after the reaction was detected to be finished by TLC ($CH_2Cl_2$:$CH_3OH$=25:1), a small amount of 10% sodium sulfite aqueous solution was used to terminate the reaction, and the reaction system was cooled to -15 °C and then stirred to precipitate a yellow solid. After suction filtration, a filter cake was washed using cold methanol (2×5 mL) and dried, to obtain a yellow solid, namely, a target product I-2 (4.2 g, 53%).$^1$H NMR (400 MHz, DMSO) δ 7.85 (d, J=8.64 Hz, 1H), 7.66 (d, J=8.64 Hz, 1H); MS (M+$H^+$): m/z=216.97.

(2) Synthesis of compound I-3

The Compound I-**2** (1 g, 4.6 mmol) was added to a three-necked flask, and dissolved with 20 mL of ethanol. After three times of replacement with nitrogen, zinc powder (1.5 g, 22.9 mmol) and ammonium chloride (2.46 g, 46 mmol) were added thereto, and replacement with nitrogen was performed again; the reaction was carried out at 50 °C for 16 h. After the reaction was detected to be finished by TLC ($CH_2Cl_2$:$CH_3OH$=25:1), a reaction solution was filtered to collect a filtrate, and the filtrate was concentrated under reduced pressure, and then purified by column chromatography with petroleum ether/ethyl acetate (5:1) to obtain a dark gray solid, namely, a target product I-**3** (524 mg, 60%)。

**$^1$H NMR** (400 MHz, DMSO) δ 9.70 (s, 1H), 6.74 (d, J=7.8 Hz, 1H), 6.49 (d, J=7.8 Hz, 1H), 5.91 (s, 1H); MS (M+$H^+$):m/z=188.99.

(3) Synthesis of compound I-5

To a reaction flask the compound I-**3** (0.3 g, 1.59 mmol) was added, and dissolved with 10 mL of ethanol, and then carbon disulfide (2.42 g, 31.7 mmol) and potassium hydroxide (191 mg, 4.76 mmol) were added while stirring; the reaction was carried out at 80-85 °C for 16 h. After the reaction was detected to be finished by TLC ($CH_2Cl_2$:$CH_3OH$=25:1 ), ethanol and carbon disulfide were removed through concentration under reduced pressure, water was added for dissolution, and the pH of the system was adjusted with dilute hydrochloric acid to about 3, to precipitate a solid, which was filtered and dried to obtain 0.26 g of a crude product of compound I-**4**, and the next reaction was carried out directly.

To a three-necked reaction flask the compound I-**4** (0.26 g, 1.13 mmol) and potassium carbonate (156 mg, 1.13 mmol) were added, and dissolved with 10 mL of DMF. After three times of replacement with nitrogen, the reaction system was cooled to 0 °C; then 176 mg of methyl iodide (1.24 mmol) was dropwise added to the reaction flask. After dropwise addition, the mixture was stirred and reacted at 0 °C for 10 min, and then reacted at room temperature for 3 h; after the reaction was detected to be finished by TLC ($CH_2Cl_2$:$CH_3OH$=25:1 ), 60 mL of water and 20 mL of ethyl acetate were added to the reaction system, stirred for 5 min and then stood; organic phases were collected, dried with anhydrous $MgSO_4$, filtered, and concentrated under reduced pressure to remove a solvent and obtain a tan solid I-5 (180 mg, 65%). $^1$H NMR (400 MHz, DMSO) δ 8.06 (d, J=8.4 Hz, 1H), 7.55 (d, J=8.4 Hz, 1H), 2.79 (s, 3H); MS (M+$H^+$):m/z=246.93.

(4) Synthesis of compound I-6

To a reaction flask 1,4-diazabicyclo[3.2.2]nonane (200 mg, 1.58 mmol), the compound I-5 (466 mg, 1.9 mmol), triethylamine (321 mg, 3.17 mmol) and 10 mL of isopropanol were added in sequence, the system was heated to 100

°C while stirring, and continued to be stirred for reaction overnight after a solvent was volatilized; after the reaction was detected to be finished by TLC (ethyl acetate/methanol=10:1), ethyl acetate (containing 1% methanol and 1‰ triethylamine) was used as eluent for purification by column chromatography to obtain a light yellow solid I-6 (250 mg, 48.8%). $^{1}H$ NMR (400 MHz, DMSO) δ 7.68 (d, J=8.12 Hz, 1H), 7.15 (d, J=8.12 Hz, 1H), 4.43-4.42 (m, 1H), 3.90 (t, J=5.56 Hz, 2H), 3.09 (t, J=5.76 Hz, 2H), 3.04-2.95 (m, 4H), 2.10-2.07 (m, 2H), 1.84-1.76 (m, 2H); MS (M+H$^{+}$):m/z=323.905.

(5) Synthesis of compound I-9

To a reaction flask the compound I-**6** (100 mg, 0.31 mmol), the compound I-**7** (70 mg, 0.314 mmol), cesium carbonate (303 mg, 0.93 mmol) and Pd(dppf)$Cl_2$ (45 mg, 0.062 mmol) were added. After the reaction system was replaced with nitrogen three times, double distilled 1,4-dioxane (10 mL) was added; the reaction was carried out at 85-90 °C, and after the reaction was finished, a solvent was removed through concentration under reduced pressure, and ethyl acetate/methanol (10:1) was used as eluent for purification by column chromatography to obtain a light yellow solid product, namely, I-**9** (89.4 mg, 85%). **$^{1}$H NMR** (400 MHz, $CDCl_3$) δ 8.72 (td, J=8.82 Hz, 2 Hz, 1H), 8.18 (dt, J=1.76 Hz, 4.32 Hz, 1H), 7.61 (dd, J=8.24 Hz, 1.32 Hz, 1H), 7.49 (d, J=8.2 Hz, 1H), 7.30 (td, J=5.94 Hz, 2.04 Hz, 1H), 4.61 (s, 1H), 3.99 (m, 2H), 3.21-3.13 (m, 4H), 3.06-2.99 (m, 2H), 2.18-2.16 (m, 2H), 1.88-1.80 (m, 2H); **$^{13}$C NMR** (100 MHz, $CDCl_3$) δ 163.54, 161.83, 159.45, 158.78, 146.72, 146.02, 141.72, 141.05, 122.63, 122.37, 122.01, 116.37, 114.92, 56.96, 50.73, 46.32, 44.33, 26.82; MS (M+H$^{+}$):m/z=340.15.

Example 2: Synthesis of

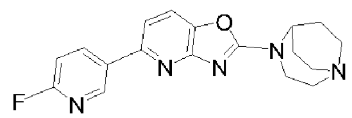

(called as compound I-10 for short) . Reference example

A synthetic route of the compound I-10 was as follows:

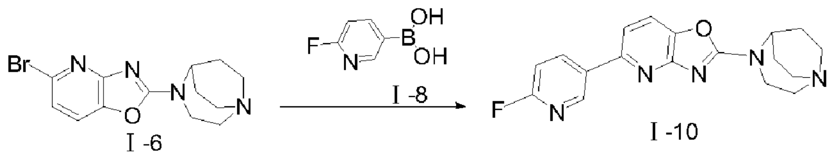


To a reaction flask the compound I-**6** (100 mg, 0.31 mmol), the compound I-**8** (44 mg, 0.313 mmol), cesium carbonate (303 mg, 0.93 mmol) and Pd(dppf)$Cl_2$ (45 mg, 0.062 mmol) were added. After the reaction system was replaced with nitrogen three times, a double distilled 1,4-dioxane (10 mL) was added; the reaction was carried out at 85-90 °C, after the reaction was finished, a solvent was removed through concentration under reduced pressure, and ethyl acetate/methanol (10:1) was used as eluent for purification by column chromatography to obtain a light yellow solid product I-10 (53 mg, 50%). **$^{1}$H NMR** (400 MHz, $CDCl_3$) δ 8.80 (d, J=2.32 Hz, 1H), 8.52 (td, J=7.8 Hz, 2.52 Hz, 1H), 7.50 (d, J=8.16 Hz, 1H), 7.35 (d, J=8.12 Hz, 1H), 6.99 (dd, J=8.52 Hz, 2.8 Hz, 1H), 4.65 (s, 1H), 4.03 (t, J=5.36 Hz, 2H), 3.25-3.19 (m, 4H), 3.10-3.04 (m, 2H), 2.22 (m, 2H), 1.93-1.86 (m, 2H); **$^{13}$C-NMR** (100 MHz, $CDCl_3$) δ 164.88, 163.48, 162.49, 158.88, 149.03, 145.90, 141.07, 139.96, 133.43, 115.37, 112.24, 109.51, 109.14, 55.83, 50.57, 46.30, 26.43, 8.70; MS (M+H$^{+}$):m/z=340.15.

Example 3: Synthesis of compound

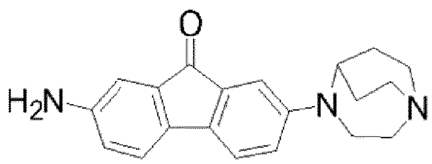

(called as compound II-5 for short)

A synthetic route of the compound II-5 was as follows:

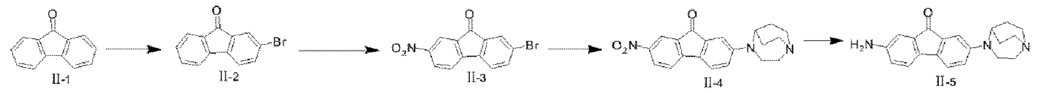

(1) Synthesis of compound II-2

To a 1000 mL reaction flask 30.0g of 9-fluorenone (II-1) (0.17 mol) and 100 mL of water were added, and heated to 80-85 °C, then bromine (32.0 g, 0.2 mol) was dropwise added to the reaction flask within 30 min. After dropwise addition, the reaction was continued at 80-85 °C for 4 h; after the reaction system was cooled to room temperature, 300 mL of water was added thereto to quench the reaction, then 300 mL of 10% NaHSOs solution was added thereto, and after 30 min of stirring, light yellow solid substances were obtained by suction filtration; the light yellow solid substances were dissolved in 300 mL of ethanol, then refluxed and stirred at 80 °C for about 16 h, cooled to room temperature and underwent suction filtration to obtain a light yellow solid, which was dried in vacuum at 50 °C to obtain 2-bromo-9-fluorenone (II-2) (39.1 g, 90.4%). **$^1$H NMR** (400 MHz, $CDCl_3$) δ 7.76 (d, J=1.76 Hz, 1H), 7.66 (d, J=7.32 Hz, 1H), 7.61 (dd, J=7.88 Hz, 1.76 Hz, 1H), 7.51-7.50 (m, 2H), 7.39 (d, J=7.88 Hz, 1H), 7.34-7.30 (m, 1H); MS ($M+H^+$):m/z=260.98.

(2) Synthesis of compound II-3

To a 1000 mL reaction flask 23.4 g of 2-bromo-9-fluorenone (II-**2**) (0.09 mol) and 180 mL of water were added, and heated to 80-85 °C, and then a mixed solution of 180 mL of 65% nitric acid solution (2.6 mol) and 180 mL of 98% sulfuric acid solution (3.3 mol) was dropwise added to the reaction system within about 30 min; the reaction system was heated to 110-120 °C, refluxed for 4 h, and after being cooled to room temperature, 300 mL of water was added thereto to quench the reaction, and obtain a yellow solid through filtration; after the obtained solid was washed with water (3×100 mL), 200 mL of methanol was added for beating, washing, and filtering to collect a solid, which was then dried in vacuum at 50 °C to obtain 2-bromo-7-nitro-9-fluorenone (II-**3**) (21.0 g, 76.4%). **$^1$H NMR** (400 MHz, $CDCl_3$) δ 8.49 (s, 1H), 8.44 (d, J=8.16 Hz, 1H), 7.90 (s, 1H), 7.75 (d, J=7.92 Hz, 1H), 7.71 (d, J=8.16 Hz, 1H), 7.55 (d, J=7.92 Hz, 1H); MS ($M+H^+$):m/z=306.02.

(3) Synthesis of compound II-4

To a 500 mL reaction flask 2-bromo-7-nitro-9-fluorenone (II-**3**) (9.0 g, 0.03 mol), $Pd_2(dba)_3$ (0.6 g, 0.655 mmol), BINAP (1.3 g, 2.087 mmol), sodium tert-butoxide (3.3 g, 0.0343 mol), 1,4-diazabicyclo[3.2.2]nonane (3.0 g, 0.024 mol) and 1,4-dioxane (150 mL) were added in sequence. After three times of replacement with nitrogen, the reaction system was heated to 80-85 °C, and the reaction was carried out under nitrogen for 16 h; after the reaction was finished, 150 mL of ethyl acetate was added, and then insoluble substances were removed by filtration through a funnel lined with diatomite; 10% $K_2CO_3$ solution was added to a filtrate to adjust the pH to about 8.0, then the filtrate was extracted with $CH_2Cl_2$ until an aqueous phase was colorless, and organic phases were combined, dried with anhydrous sodium sulfate, filtered, and concentrated under reduced pressure to obtain a bluish purple product (II-**4**) (2.1 g, 20.4%). This product could be directly used for next reaction. **$^1$H NMR** (400 MHz, $CDCl_3$) δ 7.56 (d, J=7.24 Hz, 1H), 7.38 (d, J=6.56 Hz, 1H), 7.34 (d, J=2.8 Hz, 1H), 7.14-7.09 (m, 2H), 6.78 (d, J=8.16 Hz, 1H), 4.13-4.11 (m, 1H), 3.67-3.65 (m, 1H), 3.61-3.58 (m, 1H), 3.15-3.14 (m, 4H), 3.04 (m, 2H), 2.14 (m, 2H), 1.78 (m, 2H); MS ($M+H^+$):m/z=350.16.

(4) Synthesis of compound II-5

To a reaction flask the compound II-**4** (2.1 g, 6.02 mmol), iron powder (1.7 g, 0.03 mol), ethanol (80 mL), water (54 mL) and concentrated hydrochloric acid (1.8 mL) were added in sequence, and heated to 80 °C, and the reaction of raw materials was detected to be completed by TLC (about 5 h); the pH value of the reaction system was adjusted to about 8.0 with 10% $K_2CO_3$ solution, 150 mL of ethyl acetate was added, and the mixture was filtered by a funnel lined with diatomite, a filter cake was washed with ethyl acetate, organic phases were combined, a solvent was removed through concentration under reduced pressure, and then the solid was dissolved in $CH_2Cl_2$, and washed by adding water. Organic phases were obtained through separation, dried with anhydrous $Na_2SO_4$, filtered, and concentrated under reduced pressure to obtain a final product II-**5** (1.5 g, 80%). **$^1$H NMR** (400 MHz, $CDCl_3$) δ 7.12 (d, J=8.24 Hz, 1H), 7.08 (d, J=7.92 Hz, 1H), 7.01 (d, J=2.44 Hz, 1H), 6.88 (d, J=2.2 Hz, 1H), 6.70 (dd, J=8.24 Hz, 2.52 Hz, 1H), 6.64 (dd, J=7.88 Hz, 2.24 Hz, 1H), 4.01 (m, 2H), 3.51-3.48 (m, 2H), 3.13-3.06 (m, 4H), 3.01-2.94 (m, 2H), 2.12-2.05 (m, 2H), 1.75-1.67 (m, 2H); **$^{13}$C NMR** (101 MHz, $CDCl_3$) δ 194.18, 148.14, 144.99, 135.02, 134.79, 134.56, 132.85, 119.00, 118.94, 117.11, 110.32, 108.94, 55.59, 50.91, 45.26, 43.25, 25.50; MS ($M+H^+$):m/z=320.0.

Example 4: Synthesis of compound

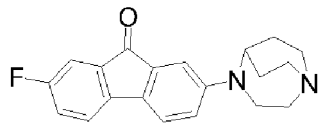

(called as compound II-6 for short)

A synthetic route of the compound II-6 was as follows:

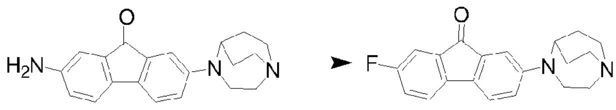

II-5 II-6

To a reaction flask the compound II-**5** (1.9 g, 6 mmol) and tetrafluoroboric acid (30 mL) were added, and the reaction flask was placed in an ice bath, to cool the reaction system to 0-5 °C, and then a sodium nitrite aqueous solution (0.5 g, 7.2 mmol, 15 mL) was dropwise added to the reaction system within 15 min; after the dropwise addition was finished, the reaction was continued for 30 min, then the stirring was stopped, and suction filtration was carried out. A filter cake was washed first with 15 mL of ethanol, then washed with 15 mL of methyl tert-butyl ether, finally beaten and washed with methyl tert-butyl ether, and filtered to obtain a solid; the solid was transferred to a reaction flask, and heated to 120 °C. TLC detection was carried out, and after the raw materials were completely converted, the reaction system was cooled to room temperature, 50 mL of $CH_2Cl_2$ was added for dissolution, the mixture was filtered to remove an insoluble solid, and a filter cake was beaten and washed with 50 mL of $CH_2Cl_2$. Organic phases were collected, dried with anhydrous $Na_2SO_4$, and then filtered, and concentrated under reduced pressure to remove a solvent; a crude product was purified by column chromatography using dichloromethane and methanol for gradient elution ($CH_2Cl_2$:$CH_3OH$=25:1) to obtain a purple solid, namely, a final product II-6 (1.2 g, 67.3%). **$^1$H NMR** (400 MHz, $CDCl_3$) $\delta$ 7.24-7.19 (m, 3H), 7.05 (d, J=2.28 Hz, 1H), 7.02 (dd, J=8.4 Hz, 2.4 Hz, 1H), 6.74 (dd, J=8.32 Hz, 2.56 Hz, 1H), 4.0437-4.0391 (m, 1H), 3.55-3.52 (m, 2H), 3.15-3.07 (m, 4H), 3.01-2.94 (m, 2H), 2.12-2.05 (m, 2H), 1.77-1.68 (m, 2H); **$^{13}$CNMR** (101 MHz, $CDCl_3$) $\delta$ 192.51 (d, J=2.2 Hz), 162.49 (s), 160.03 (s), 148.88 (s), 140.55 (d, J=2.9 Hz), 130.56 (s), 120.13 (s), 119.86 (s), 119.63 (s), 118.92 (s), 118.85 (s), 116.74 (s), 110.86 (s), 110.63 (s), 108.67 (s), 55.97 (s), 50.73 (s), 45.48 (s), 43.49 (s), 25.76 (s); **$^{19}$F NMR** (376 MHz, $CDCl_3$) $\delta$ -115.21 (s); MS ($M+H^+$):m/z=323.2.

Example 5: Synthesis of compound

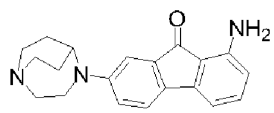

(called as compound II-14 for short)

A synthetic route of the compound II-14 was as follows:

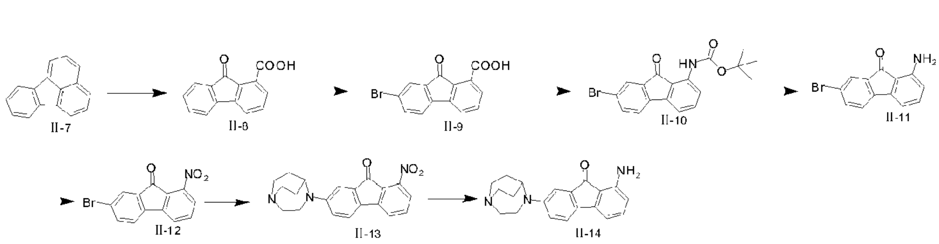

II-7 II-8 II-9 II-10 II-11 II-12 II-13 II-14

(1) Synthesis of compound II-8

Chromium trioxide (138.0 g, 1.38 mol) was dissolved in a mixed solution of 120 mL of water and 80 mL of acetic acid, and stirred to completely dissolve for later use; to a reaction flask 40.0 g of fluoranthene (II-7) (0.2 mol) and 500 mL of acetic acid were added, and the reaction system was heated to 80-85 °C, then a chromium trioxide solution was dropwise added thereto, the temperature of the system was controlled to be 80-85 °C. After the dropwise addition was completed, the reaction system was heated to 110-120 °C, cooled to room temperature after 2 h of reaction, and a reaction solution was poured into 3L of water, a large amount of yellow solid precipitated. After suction filtration, the solid

was dissolved in 600 mL of 2 M NaOH solution, underwent suction filtration to remove insoluble impurities, and a filter cake was washed with 500 mL of water; methyl tert-butyl ether was added to wash an aqueous phase, and the aqueous phase was obtained through separation. The pH of the aqueous phase was adjusted to about 1.0 using concentrated hydrochloric acid, a yellow solid precipitated again, and was dried in vacuum at 60 °C after suction filtration to obtain a target product II-**8** (9-fluorenone-1-carboxylic acid) (29.5 g, 66.0%). **$^1$H NMR** (400 MHz, $CDCl_3$) δ 8.18 (d, J=7.52 Hz, 1H), 7.74-7.53 (m, 5H), 7.36 (t, J=7.16 Hz, 1H); MS ($M+H^+$):m/z=225.10.

(2) Synthesis of compound II-9

To a reaction flask the 9-fluorenone-1-carboxylic acid (II-**8**) (15.0 g, 0.067 mol) and 300 mL of water were added. The reaction system was heated to 80-85 °C, then 10 mL of $Br_2$ (0.23 mol) was dropwise added thereto. After the dropwise addition was completed, the reaction was continued for 16 h. After the reaction was detected to be completed by TLC ($CH_2Cl_2$:$CH_3OH$=10:1), 300 mL of 10% sodium bisulfite aqueous solution was added thereto, followed by stirring for 30 min, to obtain a yellow solid upon filtration. The solid was dried in vacuum at 50 °C to obtain a final product II-**9** (19.5 g, 96.5%). **$^1$H NMR** (400 MHz, $CDCl_3$) δ 8.22 (d, J=7.04 Hz, 1H), 7.85 (s, 1H), 7.73-7.66 (m, 3H), 7.43 (d, J=7.84 Hz, 1H).

(3) Synthesis of compound II-10

Under a condition of stirring, to a reaction flask the II-**9** (6 g, 19.87 mmol), toluene (60 mL), triethylamine (4.1 mL, 29.8 mmol), DPPA (6.4 mL, 29.8 mmol) and tert-butanol (10 mL) were added in sequence, then the reaction system was heated to 110 °C and underwent reflux reaction. After the reaction was detected to be finished by TLC (raw materials, $CH_2Cl_2$:$CH_3OH$=5:1; intermediate, petroleum ether:ethyl acetate=5:1), a solvent was removed, followed by purification with silica gel column (petroleum ether: ethyl acetate was 30:1) to obtain a yellow solid, namely a product II-**10** (5.5 g, 74%). **$^1$H NMR** (400 MHz, $CDCl_3$) δ 8.15 (d, J=8.56 Hz, 1H), 7.71 (d, J=1.72 Hz, 1H), 7.59 (dd, J=7.88 Hz, 1.8 Hz, 1H), 7.42 (dd, J=7.44 Hz, 8.36 Hz, 1H), 7.37 (d, J=7.92 Hz, 1H), 7.09 (d, J=7.16 Hz, 1H), 1.55 (s, 9H).

(4) Synthesis of compound II-11

The compound II-**10** (808 mg, 2.16 mmol) was dissolved in 40 mL of acetonitrile, to which 22 mL of 1 M hydrochloric acid (21.6 mmol) was added, and heated to react at 82 °C for 4 h. After the reaction was detected to be finished by TLC, the reaction system was cooled to room temperature, a proper amount of 1 M NaOH solution was added to the reaction system to adjust the pH to be strong alkaline, followed by extraction with ethyl acetate, to collect organic phases, which were dried with anhydrous sodium sulfate, filtered, and concentrated under reduced pressure to remove a solvent and obtain a yellow solid, namely a product II-**11** (500 mg, 84.5%). **$^1$H NMR** (400 MHz, $CDCl_3$) δ 7.71 (d, J=1.4 Hz, 1H), 7.55 (dd, J=7.8 Hz, 1H), 7.35 (d, J=7.9 Hz, 1H), 7.22 (t, J=7.6 Hz, 1H), 6.81 (d, J=7.1 Hz, 1H), 6.52 (d, J=8.4 Hz, 1H), 5.54 (s, 2H); MS ($M+H^+$):m/z=273.99.

(5) Synthesis of compound II-12

To a 25 mL three-neck flask 1.729 mL of 30% hydrogen peroxide (16.95 mmol) was added. After the three-neck flask was cooled to 0 °C in an ice bath, a dichloromethane (2 mL) solution of trifluoroacetic anhydride (2.736 mL, 19.68 mmol) was dropwise added thereto in argon ambient. After the dropwise addition was completed, the reaction system continued to be stirred at 0 °C for 1.5 h (the reaction mixture turned brown from colorless); then a dichloromethane (4 mL) solution of the compound II-**11** (500 mg, 1.824 mmol) was dropwise added to the reaction system, and they were heated to room temperature after the dropwise addition, followed by stirring and reaction for 1 h. After the reaction was detected to be finished by TLC, a proper amount of water was added to quench the reaction, followed by extraction with dichloromethane, to collect organic phases, which were dried with anhydrous sodium sulfate, filtered, and subjected to rotary evaporation under reduced pressure to remove a solvent, followed by silica gel column chromatographic purification (petroleum ether:ethyl acetate=10:1) to obtain a yellow solid II-**12** (212 mg, 38.2%). **$^1$H NMR** (400 MHz, $CDCl_3$) δ 8.32 (d, J=8.4 Hz, 1H), 7.75 (d, J=1.8 Hz, 1H), 7.64 (dd, J=7.9 Hz, 1H), 7.54 (t, J=7.6 Hz, 1H), 7.41 (d, J=7.9 Hz, 1H), 7.31 (d, J=7.4 Hz, 1H).

(6) Synthesis of compound II-13

The compound II-**12** (100 mg, 0.329 mmol), 1,4-diazabicyclo[3.2.2]nonane (125 mg, 0.989 mmol) and cesium carbonate (426 mg, 1.31 mmol) were dissolved in 2 mL of double distilled anhydrous toluene for later use; $Pd_2(dba)_3$ (30 mg, 0.033 mmol) and (±)-BINAP (61 mg, 0.099 mmol) were dissolved in 2 mL of double distilled anhydrous toluene

under argon. After being stirred at 90 °C for 15 min (the reaction mixture turned from a dark purple turbid liquid to an orange yellow clear liquid), the mixture was cooled to room temperature; then the preceding solution was added to the reaction system, after being stirred at 60 °C for 14 h (the reaction mixture turned from the orange yellow clear liquid to a brownish red liquid) under argon, the reaction system was cooled to room temperature, the reaction was quenched by adding 10 mL of water, followed by extraction with dichloromethane to collect organic phases, which were dried with anhydrous sodium sulfate, filtered, and subjected to rotatory evaporation under reduced pressure to remove a solvent, followed by silica gel column chromatographic purification ($CH_2Cl_2$: $CH_3OH$=20:1) to obtain a purple black solid II-**13** (48 mg, 41.8%). **$^1$H NMR** (400 MHz, $CDCl_3$) $\delta$ 7.49-7.52 (m, 2H), 7.36 (d, J=7.9 Hz, 2H), 7.10 (s, 1H), 6.82 (t, J=7.6 Hz, 1H), 4.13 (s, 1H), 3.62-3.63 (m, 2H), 3.04-3.18 (m, 6H), 2.31 (s, 2H), 1.82-1.83 (m, 2H); MS (M+H$^+$):m/z=350.37.

(7) Synthesis of compound II-14

To a reaction flask the II-**12** (2.1 g, 6.02 mmol), iron powder (1.7 g, 0.03 mol), ethanol (80 mL), water (54 mL) and concentrated hydrochloric acid (1.8 mL) were added in sequence, and heated to 80 °C to react for 5 h; after the reaction of the raw materials was detected to be completed by TLC, the pH value was adjusted to about 8.0 using 10% $K_2CO_3$ solution, 150 mL of ethyl acetate was added, followed by filtration through a funnel lined with diatomite, combination of organic phases, and removal of a solvent through concentration under reduced pressure, then the solid was dissolved in $CH_2Cl_2$, and washed by adding water, followed by separation to obtain the organic phases, which were dried with anhydrous $Na_2SO_4$, filtered, and concentrated under reduced pressure to obtain a final product II-**14** (1.5 g, 80%). **$^1$H NMR** (400 MHz, $CDCl_3$) $\delta$ 7.30 (d, J=8.28 Hz, 1H), 7.16-7.12 (m, 1H), 7.06 (d, J=2.48 Hz, 1H), 6.75 (dd, J=8.28 Hz, 2.52 Hz, 1H), 6.67 (d, J=7.04 Hz, 1H), 6.35 (d, J=8.28 Hz, 1H), 5.43 (s, 2H), 4.10 (s, 1H), 3.58 (t, J=5.48 Hz, 2H), 3.19-3.13 (m, 3H), 3.07-3.00 (m, 2H), 2.14-2.11 (m, 2H), 1.80-1.74 (m, 3H); **$^{13}$C NMR** (100 MHz, $CDCl_3$) $\delta$ 195.66 (s), 150.20 (s), 147.18 (s), 137.02-136.73 (m), 136.37 (s), 131.22 (s), 121.42 (s), 116.55 (s), 115.39 (s), 108.58 (s), 108.48 (s), 57.06 (s), 51.87 (s), 46.56 (s), 44.64 (s), 26.91 (s); MS (M+H$^+$):m/z=320.16.

Example 6: Synthesis of compound

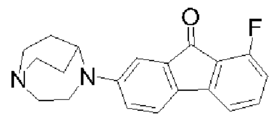

(called as compound II-15 for short)

A synthetic route of compound II-15 was as follows:

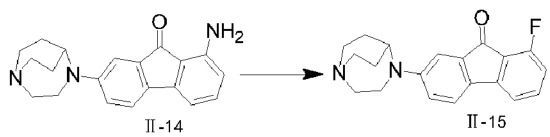

II-14 II-15

To a reaction flask the compound II-**14** (1.3 g, 4.07 mmol) and tetrafluoroboric acid (20 mL) were added. The reaction flask was placed in an ice bath, and after the temperature was reduced to 0-5 °C, a $NaNO_2$ solution (0.4 g, 5.8 mmol, 10 mL) was dropwise added thereto, and the reaction was continued for 30 min after the dropwise addition was finished; stirring was stopped, suction filtration was carried out, a filter cake was washed first with 15 mL of ethanol, then washed with 15 mL of methyl tert-butyl ether, finally beaten and washed with methyl tert-butyl ether, and filtered to obtain a solid; the solid was transferred to a reaction flask, heated to 120 °C, cooled to room temperature after the reaction was detected to be finished by TLC. 50 mL of dichloromethane was added for dissolution, followed by filtration to remove insoluble solids, the filter cake was beaten and washed again with 50 mL of dichloromethane, followed by combination of organic phases, concentration under reduced pressure to remove a solvent and obtain a crude product, which was purified by column chromatography using dichloromethane and methanol (25:1) to obtain a purple solid II-**15** (446 mg, 34.0%). **$^1$H NMR** (400 MHz, $CDCl_3$) $\delta$ 7.39-7.34 (m, 1H), 7.32 (d, J=8.36 Hz, 1H), 7.11 (d, J=7.36 Hz, 1H), 7.09 (d, J=2.48 Hz, 1H), 6.80-6.74 (m, 2H), 4.10 (s, 1H), 3.60 (t, J=5.76 Hz, 2H), 3.19-3.12 (m, 4H), 3.05-2.99 (m, 2H), 2.15-2.10 (m, 2H), 1.82-1.73 (m, 2H); **$^{13}$C NMR** (101 MHz, $CDCl_3$) $\delta$ 191.17 (s), 158.08 (s), 150.54 (s), 137.22 (s), 137.14 (s), 135.82 (s), 130.97 (s), 121.76 (s), 117.27 (s), 115.33 (s), 115.12 (s), 114.98 (s), 109.26 (s), 56.85 (s), 51.62 (s), 46.36 (s), 44.39 (s), 29.70 (s), 26.65 (s); **$^{19}$F NMR** (376 MHz, $CDCl_3$) $\delta$ -114.11 (s); MS (M+H$^+$):m/z=323.2.

Example 7: Synthesis of compound

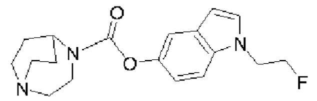

(called as compound III-5 for short) . Reference example

A synthetic route of compound III-5 was as follows:

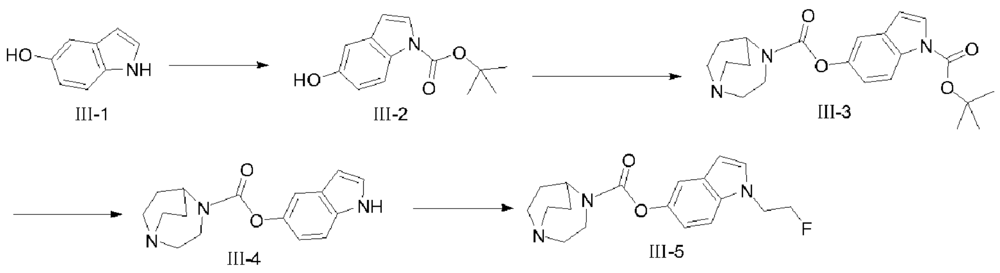

III-1 III-2 III-3 III-4 III-5

(1) Synthesis of compound III-2

5-hydroxyindole (III-1) (10 g, 75.1 mmol) was dissolved in 100 mL of acetonitrile, and di-tert-butyl dicarbonate (49.2 g, 225.3 mmol) and DMAP (917 mg, 7.51 mmol) were added to the solution, followed by stirring at room temperature for 15 h; after the reaction was finished, the reaction solution was concentrated under reduced pressure, 400 mL of methanol was added for dissolution, then $K_2CO_3$ (51.9 g, 375.5 mmol) was added, followed by stirring at room temperature for 4 h; after the reaction was completed, the pH was adjusted to be neutral using acetic acid, followed by dilution by adding $H_2O$, extraction of organic phases (ethyl acetate was used as extracting agent), removal of a solvent, and separation by column chromatography (petroleum ether:ethyl acetate=3:1) to obtain a product, i.e. the compound III-2 (12.5 g, 71%). **$^1$H NMR** (400 MHz, $CDCl_3$) $\delta$ 7.99 (d, J=6.96 Hz, 1H), 7.56 (d, J=2.84 Hz, 1H), 6.98 (d, J=2.44 Hz, 1H), 6.85-6.82 (m, 1H), 6.46 (d, J=3.64 Hz, 1H), 1.66 (s, 9H); MS (M-$H^+$):m/z=232.11.

(2) Synthesis of compound III-3

The compound III-2 (3.2 g, 13.7 mmol) was dissolved in 20 mL of $CH_2Cl_2$, diisopropylethylamine (1.77 g, 13.7 mmol) was dissolved in 120 mL of tetrahydrofuran, the two were mixed, then the mixed solution was slowly added to a $CH_2Cl_2$ (100 mL) solution of triphosgene (1.3 g, 4.38 mmol), and after stirring at room temperature for 1 h, a $CH_2Cl_2$ solution of 1,4-diazabicyclo[3.2.2]nonane (1.72 g, 13.7 mmol) was slowly added thereto, and the reaction was carried out at room temperature for 4 h; after the reaction was finished, $H_2O$ was added for dilution, followed by extraction with $CHCl_3$, collection of organic phases, which were washed with a saturated aqueous solution of NaCl, dried with anhydrous $Na_2SO_4$, to remove a solvent and obtain a crude product; separation by column chromatography was carried out with $CHCl_3$ and $CH_3OH$ (90:10) as eluents to obtain a product III-3 (2.4 g, 45%). **$^1$H NMR** (400 MHz, $CDCl_3$) $\delta$ 8.11 (d, J=7.6 Hz, 1H), 7.60 (s, 1H), 7.30 (m, 1H), 7.05 (d, J=8.96 Hz, 1H), 6.52 (d, J=3.2 Hz, 1H), 4.52-4.41 (m, 1H), 3.89 (t, J=5.48 Hz, 1H), 3.78 (t, J=5.68 Hz, 1H), 3.20-3.06 (m, 6H), 2.14-2.11 (m, 2H), 1.82-1.72 (m, 2H), 1.66 (s, 9H); MS (M+$H^+$):m/z=386.21.

(3) Synthesis of compound III-4

The compound III-3 (2.4 g, 6.23 mmol) was dissolved in 20 mL of $CH_2Cl_2$, and after the temperature of a reaction solution was reduced to 0 °C, 10 mL of trifluoroacetic acid was added thereto, followed by stirring at 30 °C for 2 h; after the reaction was finished, the reaction solution was concentrated, diluted by adding water, and extracted with $CH_2Cl_2$; the pH of an aqueous phase was adjusted to 8-9 with saturated NaHCOs solution, then extracted with $CH_2Cl_2$, dried with anhydrous sodium sulfate, to remove a solvent and obtain a product III-4 (360 mg, 20%). **$^1$H NMR** (400 MHz, DMSO) $\delta$ 11.16 (s, 1H), 7.38-7.26 (m, 3H), 6.84 (d, J=8.52 Hz, 1H), 6.40 (s, 1H), 4.53-4.33 (m, 1H), 3.95 (m, 1H), 3.79 (m, 1H), 3.30 (m, 6H), 2.20-2.10 (m, 2H), 1.95-1.93 (m, 2H); MS (M+$H^+$):m/z=286.15.

(4) Synthesis of compound III-5

At 0 °C, sodium hydride (59 mg, 1.48 mmol) was added to an anhydrous DMF (3 mL) solution of the compound III-4 (350 mg, 1.23 mmol). After 20 min of stirring, an anhydrous DMF (3 mL) solution of 1-fluoro-2-iodoethane (321 mg,

1.85 mmol) was slowly added thereto, and then the reaction was carried out at room temperature for 2 h; after the reaction was finished, an ammonium chloride solution was added thereto for dilution, followed by extraction with ethyl acetate, collection of organic phases, and purification by column chromatography using $CHCl_3$ and $CH_3OH$ (95:5, containing 20 drops of ammonia water) to obtain a target product III-5 (50 mg, 12.3%). **$^1$H NMR**

(400 MHz, $CDCl_3$) δ 7.36-7.34 (m, 1H), 7.26 (d, J=8.76 Hz, 1H), 7.14 (d, J=3.04 Hz, 1H), 6.98 (d, J=8.76 Hz, 1H), 6.48 (d, J=3.0 Hz, 1H), 4.72 (t, J=4.88 Hz, 1H), 4.61 (t, J=4.88 Hz, 1H), 4.51 (m, 1H), 4.39 (t, J=4.88 Hz, 1H), 4.32 (t, J=4.88 Hz, 1H), 3.87 (t, J=5.72 Hz, 1H), 3.76 (t, J=5.8 Hz, 1H), 3.17-3.01 (m, 6H), 2.13-2.10 (m, 2H), 1.80-1.69 (m, 2H); MS (M+H$^+$):m/z=332.19.

Example 8: Synthesis of compound

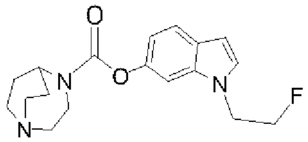

called as compound III-12 for short) Reference example

A synthetic route of compound III-12 was as follows:

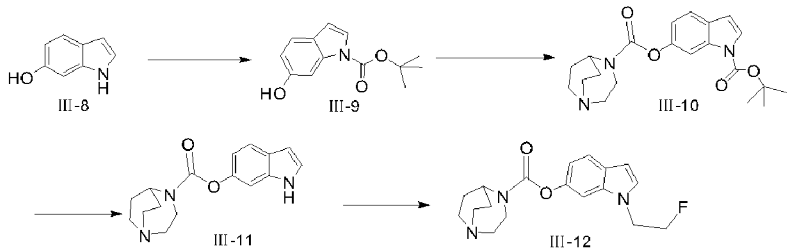

III-8 III-9 III-10 III-11 III-12

(1) Synthesis of compound III-9

6-hydroxyindole (III-8) (10 g, 75.1 mmol) was dissolved in 100 mL of acetonitrile, and di-tert-butyl dicarbonate (49.2 g, 225.3 mmol) and DMAP (917 mg, 7.51 mmol) were added to the solution, followed by stirring at room temperature for 15 h; after the reaction was finished, a reaction solution was concentrated under reduced pressure, 400 mL of methanol was added for dissolution, then $K_2CO_3$ (51.9 g, 375.5 mmol) was added, followed by stirring at room temperature for 4 h; after the reaction was completed, the pH was adjusted with $CH_3COOH$ to be neutral, followed by dilution by adding $H_2O$, and extraction to collect organic phases (ethyl acetate as extracting agent); after removal of a solvent, separation by column chromatography was carried out (petroleum ether:ethyl acetate=3:1) to obtain a product III-9 (12.5 g, 71%). **$^1$H NMR** (400 MHz, $CDCl_3$) δ 7.68 (s, 1H), 7.45 (d, J=3.16 Hz, 1H), 7.39 (d, J=8.4 Hz, 1H), 6.79 (dd, J=8.4 Hz, 2.2 Hz, 1H), 6.48 (d, J=3.68 Hz, 1H); MS (M-H$^+$): m/z=232.11.

(2) Synthesis of compound III-10

The compound III-9 (3.2 g, 13.7 mmol) was dissolved in 20 mL of $CH_2Cl_2$, diisopropylethylamine (1.77 g, 13.7 mmol) was dissolved in 120 mL of tetrahydrofuran, and the two were mixed, then the mixed solution was slowly added to a $CH_2Cl_2$ (100 mL) solution of triphosgene (1.3 g, 4.38 mmol), and after stirring at room temperature for 1 h, a $CH_2Cl_2$ solution of 1,4-diazabicyclo[3.2.2]nonane (1.72 g, 13.7 mmol) was slowly added thereto, and the reaction was carried out at room temperature for 4 h; after the reaction was completed, $H_2O$ was added for dilution, followed by extraction with $CHCl_3$ to collect organic phases, which were washed with a saturated aqueous solution of NaCl, dried with anhydrous $NaSO_4$, to remove a solvent and obtain a crude product; separation by column chromatography was carried out using $CHCl_3$ and $CH_3OH$ (90:10) as eluents to obtain a product III-10 (2.4 g, 45%). **$^1$H NMR** (400 MHz, $CDCl_3$) δ 7.97 (s, 1H), 7.54 (d, J=3 Hz, 1H), 7.50 (d, J=8.44 Hz, 1H), 7.02 (d, J= 8.44 Hz, 1H), 6.54 (d, J=3.64 Hz, 1H), 4.50-4.40 (m, 1H), 3.85 (t, J=5.64 Hz, 1H), 3.77 (t, J=5.8 Hz, 1H), 3.18-3.02 (m, 6H), 2.13-2.10 (m, 2H), 1.81-1.70 (m, 2H), 1.65 (s, 9H); MS (M+H$^+$):m/z=386.21.

(3) Synthesis of compound III-11

The compound III-10 (2.4 g, 6.23 mmol) was dissolved in 20 mL of $CH_2Cl_2$, after the reaction solution was

cooled to 0 °C, 10 mL of trifluoroacetic acid was added thereto, then followed by stirring at 30 °C for 2 h; after the reaction was finished, a reaction solution was concentrated, diluted by adding water, and extracted with $CH_2Cl_2$; an aqueous phase was adjusted to pH 8-9 with a saturated solution of NaHCOs, then extracted with $CH_2Cl_2$, dried with anhydrous $NaSO_4$, to remove a solvent and obtain a product III-11 (360 mg, 20%). **$^1$H NMR** (400 MHz, $CDCl_3$) $\delta$ 8.27 (s, 1H), 7.57 (d, J=8.52 Hz, 1H), 7.17 (d, J=2.36 Hz, 1H), 6.87 (d, J=8.48 Hz, 1H), 6.52 (m, 1H), 4.54-4.44 (m, 1H), 3.90 (t, J=5.72 Hz, 1H), 3.80 (t, J=5.72 Hz, 1H), 3.22-3.08 (m, 1H), 2.17-2.13 (m, 2H), 1.84-1.74 (m, 2H); MS ($M+H^+$):m/z=286.16.

(4) Synthesis of compound III-12

At 0 °C, sodium hydride (59 mg, 1.48 mmol) was added to an anhydrous DMF (3 mL) solution of the compound III-11 (350 mg, 1.23 mmol), after 20 min of stirring, an anhydrous DMF (3 mL) solution of 1-fluoro-2-iodoethane (321 mg, 1.85 mmol) was slowly added thereto, and then reaction was carried out at room temperature for 2 h; after the reaction was finished, $NH_4Cl$ solution was added thereto for dilution, followed by extraction with ethyl acetate, to collect organic phases, and purification by column chromatography using $CHCl_3$ and $CH_3OH$ (95:5, containing 20 drops of aqueous ammonia) to obtain a target product III-12 (50 mg, 12.3%). **$^1$H NMR** (400 MHz, $CDCl_3$) $\delta$ 7.57 (d, J=8.48 Hz, 1H), 7.13-7.11 (m, 2H), 6.90-6.87 (m, 1H), 6.51 (d, J=3.04 Hz, 1H), 4.75 (t, J=4.84 Hz, 1H), 4.63 (t, J=4.92 Hz, 1H), 4.51 (m, 1H), 4.38 (t, J=4.92 Hz, 1H), 4.31 (t, J=4.88 Hz, 1H), 3.87 (t, J=5.64 Hz, 1H), 3.77 (t, J=5.68 Hz, 1H), 3.18-3.01 (m, 6H), 2.13-2.10 (m, 2H), 1.81-1.69 (m, 2H); MS ($M+H^+$):m/z=332.18.

Example 9: Synthesis of compound

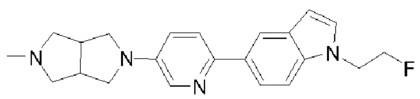

(called as compound III-19 for short). Reference example

A synthetic route of the compound III-19 was as follows:

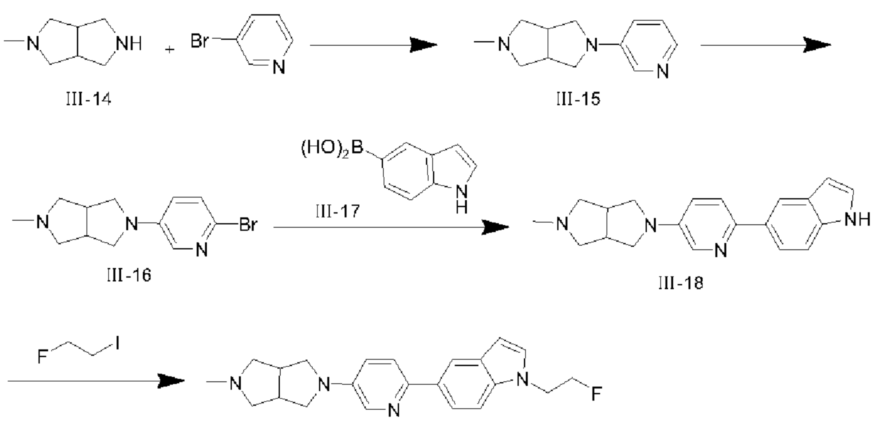
III-14
III-15
III-17
III-16
III-18

III-19

(1) Synthesis of compound III-15

The compound III-14 (2 g, 15.85 mmol), 3-bromopyridine (3 g, 18.99 mmol), $Pd_2(dba)_3$ (320 mg, 0.35 mmol), rac-BINAP (660 mg, 1.06 mmol) and sodium tert-butoxide (1.83 g, 19.04 mmol) were dissolved in 20 mL of toluene, and heated to react at 85 °C for 16 h; after the reaction was finished, the reaction system was cooled, and concentrated under reduced pressure to remove a solvent, and a crude product was separated by column chromatography using $CH_2Cl_2$:$CH_3OH$ (0.1% $NH_3 \cdot H_2O$)=10:1 to obtain a brown solid, namely, a product III-15 (2.2g, 68%). **$^1$H NMR** (400 MHz, $CDCl_3$) $\delta$ 8.05 (d, J=2.4 Hz, 1H), 7.98 (d, J=4.52 Hz, 1H), 7.12-7.09 (m, 1H), 6.90 (d, J=8.28 Hz, 1H), 3.43-3.39 (m, 2H), 3.23-3.20 (m, 2H), 3.0025-2.9960 (m, 2H), 2.76-2.72 (m, 2H), 2.50-2.47 (m, 2H), 2.34 (s, 3H); MS ($M+H^+$):m/z=204.15.

(2) Synthesis of compound III-16

At 0 °C, the compound III-15 (2.2 g, 10.82 mmol) was dissolved in 80 mL of acetonitrile, then an acetonitrile (10 mL) solution of N-bromosuccinimide (1.93 g, 10.82 mmol) was dropwise added thereto within 30 min, followed by

30 min of stirring, and then the reaction system was heated to room temperature; the reaction was quenched with 40 mL of water, $CH_2Cl_2$ (2x40 mL) was added for extraction, organic phases were collected, washed with a saturated solution of NaCl, dried with anhydrous $NaSO_4$, concentrated under reduced pressure to remove a solvent, and then a crude product was purified by column chromatography with $CH_2Cl_2$:$CH_3OH$ (0.1% $NH_3 \cdot H_2O$)=15: 1 to obtain a light brown solid, namely, a target product III-16 (1.4 g, 46%). **$^1$H NMR** (400 MHz, $CDCl_3$) δ 7.74 (s, 1H), 7.23 (d, J=8.68 Hz, 1H), 6.78 (d, J=8.68 Hz, 1H), 3.43-3.39 (m, 2H), 3.18-3.16 (m, 2H), 3.00 (m, 2H), 2.69-2.66 (m, 2H), 2.54-2.51 (m, 2H), 2.34 (s, 3H); MS ($M+H^+$):m/z=282.06.

(3) Synthesis of compound III-18

The compound III-16 (1.35 g, 4.78 mmol), the compound III-17 (1.16 g, 7.21 mmol) and $Pd_2(dba)_3$ (553 mg, 0.48 mmol) were dissolved in 40 mL of 1,4-dioxane, followed by 10 min of stirring, then 10 mL of a sodium carbonate (1.93 g, 18.21 mmol) solution was added thereto; the reaction system was heated to 115 °C, and refluxed for 2 h; after the reaction was finished, the reaction system was cooled, and a mixed solvent of water/ethyl acetate (50:50) was added thereto; a solid was collected through filtration, and washed with 30 mL of ethyl acetate to obtain a light yellow solid, namely, a target product III-18 (1.0 g, 66%). **$^1$H NMR** (400 MHz, $CDCl_3$) δ 8.23 (s, 1H), 8.16 (m, 2H), 7.81 (d, J=8.48 Hz, 1H), 7.63 (d, J=8.68 Hz, 1H), 7.44 (d, J=8.52 Hz, 1H), 7.22 (s, 1H), 7.03-6.99 (m, 1H), 6.60 (s, 1H), 3.47-3.43 (m, 2H), 3.29-3.27 (m, 2H), 3.01 (m, 2H), 2.79-2.75 (m, 2H), 2.50-2.47 (m, 2H), 2.35 (s, 3H); MS ($M+H^+$):m/z=319.19.

(4) Synthesis of compound III-19

At 0 °C, 94 mg of sodium hydride (2.35 mmol) was added to an anhydrous DMF (3 mL) solution of the compound III-18 (500 mg, 1.57 mmol), followed by 30 min of stirring, then an anhydrous DMF (3 mL) solution of 1-fluoro-2 iodoethane (546 mg, 3.14 mmol) was slowly added thereto, an ice-water bath was removed and the reaction system was heated to room temperature, and reaction was carried out for 2 h; after the reaction was completed, the reaction system was diluted with $NH_4Cl$ solution, extracted by adding $CH_2Cl_2$ to collect organic phases, and separated by column chromatography with $CH_2Cl_2$:$CH_3OH$ (0.1%$NH_3H_2O$)=15:1 to obtain a target product III-19 (180 mg, 31%). **$^1$H NMR** (400 MHz, $CDCl_3$) δ 8.16 (s, 2H), 7.85 (d, J=8.6 Hz, 1H), 7.64 (d, J=8.64 Hz, 1H), 7.37 (d, J=8.64 Hz, 1H), 7.16 (d, J=2.8 Hz, 1H), 7.03-7.00 (m, 1H), 6.58 (d, J=2.88 Hz, 1H), 4.80 (t, J=4.84 Hz, 1H), 4.68 (t, J=4.84 Hz, 1H), 4.47 (t, J=4.92 Hz, 1H), 4.40 (t, J=4.8 Hz, 1H), 3.45-3.41 (m, 2H), 3.31-3.29 (m, 2H), 3.05 (m, 2H), 2.88-2.87 (m, 2H), 2.52-2.50 (m, 2H), 2.40 (s, 3H); **$^{13}$C NMR** (100 MHz, $CDCl_3$) δ 147.67, 143.13, 135.99, 135.71, 131.92, 129.22, 128.68, 121.21, 120.68, 120.28, 118.62, 109.19, 102.66, 83.21, 81.53, 63.13, 54.49, 42.37, 41.80; MS ($M+H^+$):m/z=365.21.

Example 10: Synthesis of compound

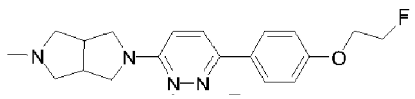

(called as compound III-24 for short) . Reference example

A synthetic route of the compound III-24 was as follows:

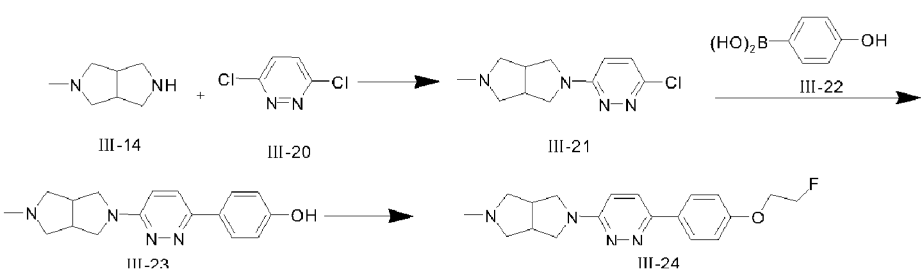


(1) Synthesis of compound III-21

2.22 mL of DIEPA (13.4 mmol) and the compound III-14 (847 mg, 6.71 mmol) were added to an n-octanol (20 ML) solution of compound III-20 (1 g, 6.71 mmol), and reaction was carried out at 120 °C overnight; after the reaction was completed, a solvent was removed through concentration under reduced pressure, and a crude product was purified by column chromatography using $CH_2Cl_2$:$CH_3OH$ (2M $NH_3 \cdot H_2O$)=4:1 as an eluent, to obtain a white solid, namely, a target product III-21 (1.2 g, 75%). **$^1$H NMR** (400 MHz, $CDCl_3$) δ 7.165 (d, J=9.36 Hz, 1H), 6.656 (d, J=9.4 Hz, 1H),

3.72-3.68 (m, 2H), 3.47 (m, 2H), 3.06 (m, 2H), 2.76-2.72 (m, 2H), 2.59-2.57 (m, 2H), 2.366 (s, 3H); MS ($M+H^+$):m/z=239.11.

(2) Synthesis of compound III-23

At room temperature, $Pd_2(dba)_3$ (421 mg, 0.46 mmol) and 1,3-bis(2,6-diisopropylphenyl)imidazolium chloride (587 mg, 1.38 mmol) were added to a mixed 1,4-dioxane (80 mL) solution of compounds III-21 (1.1 g, 4.61 mmol) and III-22 (763 mg, 5.53 mmol); after three times of replacement with nitrogen, 20% $Na_2CO_3$ (10 mL, 18.4 mmol) was added thereto, and replacement with nitrogen was carried out again (4 times); the reaction was carried out at 85 °C under nitrogen for 19 h, and after the reaction was finished, the reaction system was cooled; 200 mL of ethyl acetate was added thereto, followed by filtration with diatomite; a filtrate was collected, and a solvent was removed through concentration, and a crude product was purified by column chromatography with $CH_2Cl_2$:$CH_3OH$ (2M $NH_3{\cdot}H_2O$)=9:1 to obtain a tan solid, namely, a target product III-23 (850 mg, 62%). **$^1$H NMR** (400 MHz, $CDCl_3$) δ 7.596 (d, J=8.2 Hz, 2H), 7.231 (m, 1H), 6.722 (d, J=8.24 Hz, 2H), 6.388 (d, J=9.44 Hz, 1H), 3.72-3.70 (m, 2H), 3.68-3.59 (m, 2H), 3.093 (m, 2H), 2.88-2.86 (m, 2H), 2.67-2.63 (m, 2H), 2.407 (s, 3H); MS ($M+H^+$):m/z=297.16.

(3) Synthesis of compound III-24

$K_2CO_3$ (652 mg, 4.72 mmol) and the compound III-23 (400 mg, 1.35 mmol) were added in sequence to an N,N-dimethylformamide (10 mL) solution of 1-fluoro-2-iodoethane (351 mg, 2.02 mmol); the mixture was stirred at room temperature overnight, and after the reaction was completed, water and ethyl acetate were added thereto, to separate organic phases and an aqueous phase. The aqueous phase was extracted twice with ethyl acetate, and the organic phases were collected, dried with anhydrous $Na_2SO_4$, and filtered to remove a solvent; a crude product was separated by column chromatography with $CH_2Cl_2$:$CH_3OH$ (2M $NH_3{\cdot}H_2O$)=9:1 to obtain a target product III-24 (160 mg, 34.6%). **$^1$H NMR** (400 MHz, $CDCl_3$) δ 7.947 (d, J=8.68 Hz, 2H), 7.587 (d, J=9.4 Hz, 1H), 7.014 (d, J=8.72 Hz, 2H), 6.751 (d, J=9.4 Hz, 1H), 4.844 (t, J=3.92 Hz, 1H), 4.726 (t, J=4.08 Hz, 1H), 4.304 (t, J=3.96 Hz, 1H), 4.234 (t, J=4.16 Hz, 1H), 3.76-3.72 (m, 2H), 3.60-3.57 (m, 2H), 3.116 (m, 2H), 2.96-2.90 (m, 2H), 2.60-2.58 (m, 2H), 2.431 (s, 3H); **$^{13}$C NMR** (100 MHz, $CDCl_3$) δ 158.21, 148.87, 139.36, 133.07, 121.35, 120.55, 120.09, 118.33, 110.13, 82.70, 81.01, 63.01, 54.79, 42.25, 41.70; MS ($M+H^+$):m/z=343.19.

Example 11: Synthesis of radioligand

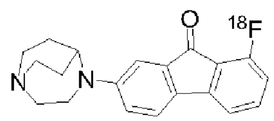

(called as [$^{18}$F] II-**15** for short)

A synthetic route of [$^{18}$F] II-**15** was as follows:

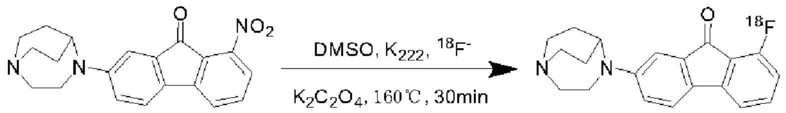


II-13

[$^{18}$F] II-15

15 mg of Kryptofix 222 was dissolved in 0.7 mL of anhydrous acetonitrile, 2 mg of $K_2C_2O_4$ was dissolved in 0.3 mL of water, and then the two were uniformly mixed to prepare 1.0 mL of Kryptofix 222/$K_2C_2O_4$ eluting solution; $^{18}F^-$ captured on a QMA column was eluated into a reaction flask using the eluting solution, the solvent in the reaction flask was dried by blowing with a flow of $N_2$ at 100 °C, then 0.5 mL of anhydrous acetonitrile was added thereto, the mixture was dried by blowing again. This process was repeated for three times to ensure that moisture in the reaction flask was fully removed; an anhydrous DMSO solution (0.3 mL) of II-13 to be labled (2 mg) was quickly added to the above reaction flask, sealed, and reacted at 160 °C for 30 min; after the reaction was finished, distilled water (2x10 mL) was added to quench the reaction, and a reaction solution was sucked by a syringe so as to pass through a Sep-Pak C18 solid phase extraction column activated in advance; then, a reaction product was eluted from the C18 column with 2 mL of acetonitrile, the elutate was collected, and concentrated under reduced pressure to remove a solvent, after a proper amount of acetonitrile was added for dissolution, radio-HPLC separation purification was carried out with acetonitrile:water (containing 0.2% of ammonium acetate)=28:72 as mobile phase, at a flow rate of 4 mL/min, and a wavelength of 280 nm. A semi-preparative column was Inertsil® ODS-3 type C18 reverse phase semi-preparative column (GL Sciences, Inc. 5

μm, 10 mm × 250 mm).

After radio-HPLC separation purification, the radioligand [$^{18}$F] II-**15** had a radiochemical purity of greater than 98%, and a radiolabeling rate of about 13.1% (uncorrected for decay); purified [$^{18}$F] II-**15** and unlabelled stable compound II-**15** were co-injected for HPLC analysis, with a mobile phase of acetonitrile:water (containing 0.2% of ammonium acetate)=30:70, a flow rate of 1 mL/min, a wavelength of 280 nm, an analytical column Agela Technologies, Venusil XBP C18 (L), 5 μm, 150Å, 4.6x250 mm. Analysis results are as shown in FIG. 1, where retention time of [$^{18}$F] II-**15** and II-**15** is 14.037 min and 13.466 min, respectively, thus retention time of the two is matched, confirming the accuracy of the radioligand.

Example 12: *In vitro* competition experiment of ligand compound

(1) Saturation binding experiment

a) Preparation of receptor protein and measurement of concentration thereof

All receptor proteins used in an experimental process were separated and extracted from brains of female SD rats (180-200 g). After female SD rats (180-200 g) were sacrificed by cervical dislocation, their brains were quickly taken out and placed on ice blocks, and after blood streaks were washed off with ice-cold normal saline, cerebral cortex was dissected out (this region was enriched with α7 nAChRs receptor proteins), and placed in an ice-cold 50 mM Tris-HCl buffer solution of 10-times volume (50 mM Tris, 120 mM NaCl, 5 mM KCl, 2 mM $CaCl_2$, 1 mM $MgCl_2$, pH=7.4, 4°C), and then a beaker was placed in an ice-water bath, and the mixture was homogenized by a handheld tissue homogenizer for 30 s (set as No. 6). The homogenized membrane solution was divided into three equal parts into 50 mL centrifuge tubes, centrifuged with a low-temperature high-speed centrifuge for 20 min (4 °C, 48000 g), supernatant was discarded after the centrifugation was completed, lower precipitates were dissolved in an ice-cold 50 mM Tris-HCl buffer solution of 10-times volume, and the mixture was homogenized, centrifuged and washed in the same manner. The lower precipitates obtained after repeating the steps 3 times were receptor membrane proteins, which were dissolved in ice-cold 50 mM Tris-HCl buffer solution of 10-times volume, and homogenized such that they were fully and uniformly mixed. 10 μL of the uniformly mixed receptor membrane protein solution was taken out, the concentration of the proteins was measured using a Lowry method, and the remaining membrane solution was subpackaged in 2 mL centrifuge tube, and stored in a refrigerator at -80 °C for later use.

b) Saturation binding experiment of receptor membrane protein

The saturation binding experiment was performed by measuring the binding of radioligand [$^{125}$I] α-bungarotoxin (called as [$^{125}$I] α-bgt for short) with mouse brain membrane proteins. In the experiment, [$^{125}$I] α-bgt was set up with 8 different concentration points (0.005-5 nM), each concentration point has 3 parallel groups. Acceptor membrane proteins stored in the -80 °C refrigerator were taken out, underwent freezing-thawing at 4 °C, and after freezing-thawing, an appropriate volume of ice-cold 50 mM Tris-HCl buffer solution (50 mM Tris, 120 mM NaCl, 5 mM KCl, 2 mM $CaCl_2$, 1 mM $mgCl_2$, pH 7.4, 4 °C) was added for dilution depending on the protein concentration measured. A total volume of the reaction mixture in a total binding tube was 500 μL, including 100 μL of membrane protein solution (an amount of proteins finally in each tube was 1.5 mg), 10 μL of radioligand [$^{125}$I] α-bgt of various concentrations and 390 μL of ice-cold 50 mM Tris-HCl buffer solution, loaded in a following order: membrane proteins, Tris-HCl buffer solution and [$^{125}$I] α-bgt (Table 1). Non-specific binding was determined by 2 μM unlabeled α-bgt, and the reaction mixture in the test tube included 100 μL of membrane protein solution (an amount of protein finally in each test tube was 1.5 mg), 10 μL of radioligand [$^{125}$I] α-bgt of various concentrations, 100 μL of 2 μM α-bgt and 290 μL of ice-cold 50 mM Tris-HCl buffer solution, in a total volume of 500 μL, loaded in a following order: membrane proteins, Tris-HCl buffer solution, α-bgt and [$^{125}$I] α-bgt (Table 2). After the loading was finished, the test tube was sealed with a sealing film, vortexed for several seconds to mix them well, and then placed in a 37 °C constant temperature incubator to incubate for 2.5 h. After the incubation was completed, the test tube was taken out and placed in an ice-water bath to terminate the binding between the receptor protein and the ligand, then the mixed solution was filtered with a 48-well cell harvester onto Whatman GF/B filter paper (soaked with 0.5% polyethylenimine solution for 2.5 h in advance), the filter paper was washed with 5 mL of ice-cold 50 mM Tris-HCl buffer solution 3 times, the filter paper was removed, and the filter paper sheet was cut off and placed in a PE tube for measurement, to measure the count with a γ-counter. A difference between total binding (TB) and non-specific binding (NSB) was the specific binding (SB), i.e., SB (cpm)=TB (cpm)-NSB (cpm).

Table 1 Table for Loading to Total Binding Tube in Saturation Binding Experiment

| No. of Test Tube | Protein (μL) | [$^{125}$I]α-bgt (nM) | (μL) Tris-HCl (μL) | Total Volume (μL) |
|---|---|---|---|---|
| 1 | 100 | 10 (0.005) | 390 | 500 |
| 2 | 100 | 10 (0.04) | 390 | 500 |
| 3 | 100 | 10 (0.14) | 390 | 500 |
| 4 | 100 | 10 (0.43) | 390 | 500 |
| 5 | 100 | 10 (0.70) | 390 | 500 |
| 6 | 100 | 10 (1.38) | 390 | 500 |
| 7 | 100 | 10 (3.54) | 390 | 500 |
| 8 | 100 | 10 (5.00) | 390 | 500 |

Table 2 for Loading to Non-specific Binding Tube in Saturation Binding Experiment

| No. of Test Tube | Protein (μL) | [$^{125}$I]α-bgt (μL) (nM) | α-bgt (μL) | Tris-HCl (μL) | Total Volume (μL) |
|---|---|---|---|---|---|
| 1 | 100 | 10 (0.005) | 100 | 290 | 500 |
| 2 | 100 | 10 (0.04) | 100 | 290 | 500 |
| 3 | 100 | 10 (0.14) | 100 | 290 | 500 |
| 4 | 100 | 10 (0.43) | 100 | 290 | 500 |
| 5 | 100 | 10 (0.70) | 100 | 290 | 500 |
| 6 | 100 | 10 (1.38) | 100 | 290 | 500 |
| 7 | 100 | 10 (3.54) | 100 | 290 | 500 |
| 8 | 100 | 10 (5.00) | 100 | 290 | 500 |

c) Experimental results

In the saturation binding experiment, 8 different concentrations (0.005-5 nM) were set for the radioligand [$^{125}$I] α-bgt, and 3 groups was measured in parallel for each concentration. Experimental results showed that in the measured concentration range, specific binding of [$^{125}$I] α-bgt with the receptor membrane proteins rapidly reached saturation, and a specific binding curve is as shown in FIG. 2; a Hill straight line was obtained by plotting $\log[B/(B_{max}-B)]$ against log[L] according to the specific binding curve and equation $\log[B/(B_{max}-B)]=n_H\log[L]-\log K_d$ (as shown in FIG. 3): y=1.05828x+0.08731 (R=0.99031), a slope thereof was Hill coefficient $n_H$=1.058, indicating that binding between [$^{125}$I] α-bgt and acceptor membrane proteins was a simple single-site action system.

A linear regression equation y=46.42857-1.2987x (R=1) was obtained by plotting B/F against the specific binding amount B according to the Scatchard equation of the single-site action system: $B/F=-B/K_d+B_{max}/K_d$ (as shown in FIG. 4). According to this equation, it was obtained that under the present experimental conditions, an equilibrium dissociation constant of [$^{125}$I] α-bgt was $K_d$=0.77±0.088 nM (95% confidence interval was 0.36-1.172 nM), a maximum binding amount was $B_{max}$=35.75±4.64 fmol/mg protein (95% confidence interval was 29.88-41.62 fmol/mg protein). The experimental result was consistent with data reported in the literature ($K_d$=1.5±0.7 nM, $B_{max}$=63±17 pmol/mg protein), indicating that the measuring method used herein is credible, and can be used to measure the biological activity of the compound to be measured.

(2) Competitive binding experiment

a) Experimental method

In order to quantify the affinity of the ligand compound with α7 nAChRs, we performed *in vitro* competitive binding experiment with [$^{125}$I] α-bgt as a radioactive standard. In the experiment, the membrane protein solution (an amount of proteins in each reaction tube is 1.5 mg) was incubated together with 0.4 nM [$^{125}$I] α-bgt solution and a series of unlabeled ligand solutions of 8 different concentrations (3 groups were measured in parallel for each concentration) in a 37 °C constant temperature incubator for 2.5 h, and after the incubation was completed, the same procedure as described in the preceding saturation binding experiment and counting with a γ-counter were carried out. At the same time, in order to ensure accuracy and reliability of this experimental system, the affinity of MLA (a known highly selective, high-affinity ligand for α7 nAChRs), as a reference ligand, with α7 nAChRs in mouse brain was measured. A formulation

method and a loading method of the ligand compound are as shown in following Table 3 and Table 4:

Table 3 Formulation Method of Ligand Compound

| No. | Target Concentration (M) | Formulation Method |
|---|---|---|
| 1 | $10^{-3}$ | Weigh a certain mass of ligand compound, add 900 μL of ethanol, then add 100 μL of DMSO to help solubilization, and uniformly mixing them through vortex; (refer to the formulation of ligand MLA, and use normal saline as solvent) |
| 2 | $10^{-4}$ | Take 100 μL of the above solution, and dilute to 1 mL by adding 900 μL of ethanol; |
| 3 | $10^{-5}$ | Ibid. |
| 4 | $10^{-6}$ | Ibid. |
| 5 | $10^{-7}$ | Ibid. |
| 6 | $10^{-8}$ | Ibid. |
| 7 | $10^{-9}$ | Ibid. |
| 8 | $10^{-10}$ | Ibid. |

Notes: Table 3 is only for the formulation method of a part of compounds with a concentration ranging $10^{-3}$-$10^{-10}$ mol/L, and for other compounds with a concentration ranging, for example, $10^{-6}$-$10^{-14}$ mol/L and $10^{-5}$-$10^{-13}$ mol/L, the method of Table 3 can be adjusted slightly.

Table 4 for Loading in Competitive Binding Experiment

| No. of Test Tube | Protein (μL) | Drug (μL) | [125I]α-bgt (μL) | Tris-HCl (μL) | Total Volume (μL) |
|---|---|---|---|---|---|
| 1 | 100 | 50 | 10 | 340 | 500 |
| 2 | 100 | 50 | 10 | 340 | 500 |
| 3 | 100 | 50 | 10 | 340 | 500 |
| 4 | 100 | 50 | 10 | 340 | 500 |
| 5 | 100 | 50 | 10 | 340 | 500 |
| 6 | 100 | 50 | 10 | 340 | 500 |
| 7 | 100 | 50 | 10 | 340 | 500 |
| 8 | 100 | 50 | 10 | 340 | 500 |

Notes: A loading order was as follows: proteins, Tris-HCl buffer solution, drug (including ligand compound and MLA) and $[^{125}I]$ α-bgt.

b) Experimental results

In order to make comparison, MLA was chosen as a reference ligand, and the affinities of MLA and the designed compound to be tested to α7 nAChRs were measured simultaneously under the same experimental conditions.

In the competitive binding experiment, the reference ligand MLA and a series of compounds to be tested were set with 8 different concentrations ($10^{-6}$-$10^{-14}$ mol/L for II-14 and $10^{-5}$-$10^{-13}$ mol/L for II-5, and $10^{-3}$-$10^{-10}$ mol/L for the remaining compounds), an $IC_{50}$ value thereof was measured by inhibiting the binding of 0.4 nM $[^{125}I]$ α-bgt ($K_d$=0.77±0.088 nM) to α7 nAChRs, and respective $K_i$ values were obtained through calculation according to Cheng-Prusoff formula ($K_i=IC_{50}/(1+[L]/K_d)$). Measuring results are as shown in Table 5. Under this experimental condition, an inhibition constant $K_i$ of MLA was $K_i$=2.88±0.78 nM, substantially close to the $K_i$ value (1.09±0.09 nM) reported in the literature, indicating the feasibility of the experimental method adopted herein.

It can be seen from Table 5 that each ligand compound showed affinity to α7 nAChR membrane proteins, with the inhibition constant ($K_i$) distributed in a range of 0.005-450 nM, wherein compounds II-**14**, II-**5**, II-**15**, II-**6** and I-**9** showed quite strong inhibitory effect on $[^{125}I]$ α-bungaratoxin, of which $K_i$ values were 0.0069±0.004 nM, 0.064±0.058 nM, 2.98±1.41 nM, 7.24±1.02 nM and 21.76±1.22 nM, respectively, indicating that they have quite high affinity to α7 nAChR, especially the affinity of compound II-**14** ($K_i$=0.0069±0.004 nM) had exceeded the highest value ($K_i$=0.023 nM) of the ligand molecules of the same type currently in the world.

Table 5 *In vitro* Binding Affinity ($K_i$, nM) of **MLA** and Respective Ligand Compounds to Be Measured to α7 nAChRs[a]

| Compound | $K_i$ (nM) | Compound | $K_i$ (nM) |
|---|---|---|---|
| **MLA** | 2.88±0.78 | II-**5** | 0.064±0.058 |
| I-**9** | 21.76±1.22 | III-**5** | 364.59±149.0 1 |
| I-**10** | 377.94±118.5 34 | III-**12** | 223.55±84.78 |
| II-**15** | 2.98±1.41 | III-**19** | 455.64±152.0 0 |
| II-**14** | 0.0069±0.004 | III-**24** | 444.67±31.54 |
| II-**6** | 7.24±1.02 | | |

[a] mean of three measurements ± standard deviation

Example 13: *In vitro* hERG potassium channel inhibition experiment

a) Experimental method

At present, there are mainly three methods for measuring the inhibitory effect of a drug on the hERG potassium channel: a full-automatic patch clamp technology, a traditional patch clamp technology and FluxORTM Thallium Assay. The conventional patch clamp technology is used herein to measure the inhibitory effect of compounds on the hERG potassium channel, which method is an accepted standard method for cardiotoxicity studies and the most accurate measuring method. In the experiment, Cisapride (a compound known to have strong inhibitory effect on the hERG potassium channel) is used as a standard compound to evaluate the inhibitory effect of a ligand compound to be measured on the hERG potassium channel.

At 37 °C, an HEK293 cell line (Creacell) stably expressing hERG potassium channels was cultured (5% $CO_2$) with DMEM medium (containing 10% fetal calf serum and 0.8 mg/M1 G418); the cells were separated by TrypLE™ Express solution after subculture, then $3\times10^3$ cells were spread on a cover glass, and cultured in a 24-well plate for 18 h and then detected in the electrophysiological activity, and drug detection was started after hERG current recorded for the whole cells was stable; in the experiment, 4 concentration gradients (0.4 μM-50 μM) were set for each ligand compound to be measured, a ratio between adjacent concentrations was 5, the concentration of standard compound Cisapride ranged 1 nM-1 μM, a ratio between adjacent concentrations was 10; in the detection, each drug (including the ligand compound and the Cisapride) concentration was kept for 5 min (or was kept acting until the current was stable), and then, the next concentration was detected, the drug solution sequentially passed through the recording chamber from low concentration to high concentration by a gravity perfusion method to act on cells, to perform liquid exchange in the recording chamber; a blank control group was set before drug detection, and all experimental groups and the control group were independently and repeatedly detected 3 times.

b) Experimental results

During data processing, an action current of each concentration of the drug to be measured was calibrated first using the recorded current of a blank control group (tail current peak value of the drug to be measured / tail current peak value of the blank control), then the inhibition rate corresponding to each concentration of the drug to be measured was calculated (1-tail current peak value of the drug to be measured / tail current peak value of the blank control), and after the average value and the relative standard deviation of 3 repeated experiments were obtained, the half-inhibition concentration $IC_{50}$ value of each compound to be measured was calculated using the following equation:

$$\text{inhibition rate}=1/[1+(IC_{50}/c)^h]$$

In the above, c represents concentration of the drug to be measured, h represents Hill coefficient; fitting of the curve and the calculation of the $IC_{50}$ value were completed through assistance of IGOR software.

Results of the inhibitory effects of the standard compound Cisapride and the compounds II-**15**, II-**14**, II-**6** and II-**5** to be measured to the hERG potassium channel are as shown in Tables 6 and 7. The experimental results show that the compound II-**14** has almost no inhibitory effect ($IC_{50}$>10 μM) to the hERG potassium channel, the compound II-**5** has moderate inhibitory effect (1 μM≤$IC_{50}$≤10 μM) to the hERG potassium channel, the compounds II-**15** and II-**6** have stronger inhibitory effect (0.1 μM≤$IC_{50}$≤1 μM) to the hERG potassium channel, but their affinity ($IC_{50}$ was in the range of 460 nM-2500 nM) to the ion channel proteins is far less than their affinity to the α7 nAChR membrane protein

($IC_{50}$ value was in the range of 0.21 nM-21 nM) (see Table 8). In addition, although this measuring method is the most sensitive method for studying hERG toxicity, the inhibitory effect of the drug on hERG potassium current *in vivo* is also related to physiological factors such as concentration in blood, therefore, these ligand molecules can be further studied as potential α7 nAChR agonists.

Table 6 Inhibitory Effect of Standard Compound Cisapride to hERG Potassium Current ($IC_{50}$, nM)

| Compound | hERG Current Inhibition Proportion | | | | n | $IC_{50}$ (nM) |
|---|---|---|---|---|---|---|
| | 1 nM | 10 nM | 100 nM | 1 μM | | |
| Cisapride | 0.08±0.02 | 0.44±0.01 | 0.84±0.01 | 0.99±0.00 | 3 | 13.8±0.76nM |

Table 7 Inhibitory Effect of Compounds II-**15**, II-**14**, II-**6**, II-**5** to hERG Potassium Current ($IC_{50}$, μ M)

| Compound | hERG Current inhibition Proportion | | | | n | $IC_{50}$ (μM) |
|---|---|---|---|---|---|---|
| | 0.4 μM | 2 μM | 10 μM | 50 μM | | |
| II-**15** | 0.45±0.01 | 0.82±0.01 | 0.97±0.01 | 0.98±0.01 | 3 | 0.46±0.011 |
| II-**14** | 0.06±0.01 | 0.20±0.02 | 0.25±0.03 | 0.53±0.04 | 3 | 49.70±15.90 |
| II-**6** | 0.42±0.02 | 0.80±0.01 | 0.96±0.01 | 1 | 3 | 0.46±0.055 |
| II-**5** | 0.19±0.01 | 0.42±0.01 | 0.79±0.02 | 0.98±0.00 | 3 | 2.50±0.15 |

Table 8 Comparison of Affinity of Compounds II-**15**, II-**14**, II-**6**, II-**5** to hERG Potassium Channels and α7 nAChR

| Compound | hERG Potassium Channel $IC_{50}$ (nM) | α7 nAChR IC50 (nM) | IC50(hERG)/IC50(α7 nAChR) |
|---|---|---|---|
| II-**15** | 460 ± 11 | 6.23±2.94 | 74 |
| II-**14** | 49700 ± 15900 | 0.023±0.013 | >10000 |
| II-**6** | 460 ± 55 | 20.86±2.94 | 22 |
| II-**5** | 2500 ± 150 | 0.21±0.19 | >10000 |

Example 14: Measurement of half lethal dosage $LD_{50}$ of ligand compound **II-15**

a) Experimental method

After 60 Kunming mice (18-20 g) in total, half females and half males, were fed under experimental conditions for 3 days, they were fast but not deprived of water for 12 h, then the mice were weighed and recorded one by one, and randomly divided into 6 groups according to weights, 10 mice (half females and half males) in each group in total; 5 solutions with different concentrations and a blank control solution were formulated according to the concentration range explored in a pre-experiment, wherein a ratio of adjacent concentrations of experimental groups was 0.90-0.95, and 0.1 mL of a drug solution or a blank control solution was injected for each group of mice in an administration mode of tail vein injection; after the injection was finished, female and male mice were separately placed and fed according to concentrations, and responses of the mice were closely observed and recorded 0.5 h, 1 h, 3 h, 6 h, 12 h and 24 h after the administration (including whether behaviors were active, responses to stimulation, diet conditions, whether symptoms such as convulsion, mania, hematemesis, and teetering appeared), and dead mice were immediately dissected to observe abnormal conditions of each organ; regular observation was performed every day later, continuously for 14 days, and weights of each group of mice were recorded 2, 4, 6, 8, 10, 12 and 14 days after administration; the death rate of each group of mice was calculated during the observation period, and the half lethal dosage $LD_{50}$ of the compound to the mice was calculated based on the experimental concentration and the death rate of each group.

b) Experimental results

1. Responses of mice in the observation period

In the experiment, all the mice that died after administration had convulsion in the observation stage, and part of the mice had trembling, hematemesis, teetering and mania; dead mice were dissected out, to observe each tissue and organ of the mice, and no abnormality was found; all the administrated mice showed inappetence within 1 hour after

the administration, and then were recovered to be normal; the weights of the mice surviving in the experimental group and the mice of the blank control group were increased normally in the observation period, and the weight change of the mice of each group had no obvious difference beween experiments with different concentrations or difference between females and males. The responses of the mice during the observation period were recorded in Table 9:

Table 9 Responses of Mice in the Observation Period

| Group | Dosage (mg/kg) | Subject Animal | Number of Death | Death Rate (%) | Observation Record |
|---|---|---|---|---|---|
| 1 | 0 | 10 | 0 | 0 | Normal |
| 2 | 64.8 | 10 | 9 | 90 | Died after convulsion of about 10 s; survivors continued the convulsion, relieved after trembling, and symptoms of teetering and hematemesis appeared; |
| 3 | 60 | 10 | 7 | 70 | Died after convulsion of 6-10 min; survivors relieved after a period of convulsion; |
| 4 | 54 | 10 | 6 | 60 | Died after convulsion of 20 min; survivors relieved after a period of convulsion; |
| 5 | 48.6 | 9 | 2 | 22 | Symptoms of convulsion, teetering, and trembling appeared, and were gradually relieved; some mice died; |
| 6 | 43.74 | 10 | 1 | 10 | Symptoms of convulsion, and trembling appeared, and were gradually relieved; some mice died |

2. Calculation of half lethal dosage $LD_{50}$

There are many methods for calculating $LD_{50}$, among which the weighted probit method created by Bliss and then developed by later researchers was the most accurate and rigorous, and was the standard $LD_{50}$ calculation method recognized by researchers. This calculation method will be used herein to solve the $LD_{50}$ value of the compound to be measured to the mice.

Table 10 $LD_{50}$ Calculation Table for Compound II-**15**

| Dosage D (mg/kg) | LogD (X) | $X^2$ | n | Death Rate (%) | Probit Y | Weight Coefficient | Weight W | WX | $WX^2$ |
|---|---|---|---|---|---|---|---|---|---|
| 64.8 | 1.8116 | 3.2818 | 10 | 90 | 6.28 | 0.33589 | 3.3589 | 6.0849 | 11.0233 |
| 60 | 1.7782 | 3.1618 | 10 | 70 | 5.52 | 0.58089 | 5.8089 | 10.3291 | 18.3667 |
| 54 | 1.7324 | 3.0012 | 10 | 60 | 5.25 | 0.61609 | 6.1609 | 10.6731 | 18.4900 |
| 48.6 | 1.6866 | 2.8447 | 9 | 22 | 4.23 | 0.5026 | 4.5234 | 7.6293 | 12.8679 |
| 43.74 | 1.6409 | 2.6925 | 10 | 10 | 3.72 | 0.33589 | 3.3589 | 5.5115 | 9.0438 |
| | | | | | | | $\sum W$ | $\sum WX$ | $\sum WX^2$ |
| | | | | | | | 23.211 | 40.2280 | 69.7917 |
| Notes: weight=weight coefficient * the number of animals in each group (n); | | | | | | | | | |

The probit Y was plotted against LogD (X) to obtain an equation y=14.76x-20.53 (as shown in FIG. 5). When the death rate was 50%, the probit Y was 5.00 according to table lookups, substituting the equation to obtain x=m=LogD=1.73, and D=53.70 mg/kg.

Standard deviation: $$Sm^2 = [\frac{(m-A)^2}{\sum W(X-A)^2} + \frac{1}{\sum W}]/b^2$$, where $$\sum W(X-A)^2 = \sum WX^2 - \frac{(\sum WX)^2}{\sum W}$$; $$A = \frac{\sum WX}{\sum W}$$; b=14.76;

According to the above expression, the standard deviation was calculated to be $Sm^2$=0.0001977577, Sm=0.014;

m±1.96 Sm=1.73±0.02744, i.e., 1.70256-1.75744; the inverse logarithm was calculated respectively, and 95% confidence limit of $LD_{50}$ was taken: 50.4150-57.2058 mg/kg; thus, the $LD_{50}$ value of the compound II-**15** was 53.70 mg/kg.

As can be seen from the above process, the half lethal dosage $LD_{50}$ value of the compound II-**15** was on the order of mg/kg, which was far beyond the dosage clinically injected for one PET imaging, and therefore, the *in vivo* application of radioligand [$^{18}$F] II-**15** obtained by labeling the compound II-15 with $^{18}$F was safe.

Example 15: Measurement of [$^{18}$F] II-**15** lipid water partition coefficient

Research and development of most nerve drugs must take their ability to cross the blood brain barrier (BBB) into account, and whether drug molecules can cross the blood brain barrier is closely related to their lipid solubility. Generally, a ligand molecule is considered to be able to cross the blood brain barrier when its lipid solubility (log P) value is between 1.0 and 3.0.

The lipid water partition coefficient (log P) of [$^{18}$F] II-15 was measured in a mixed system of n-octanol and PBS of pH 7.4, and a specific experimental method was as follows: to a 10 mL centrifuge tube containing 900 μL of PBS (saturated with n-octanol in advance) and 1000 μL of n-octanol (saturated with PBS in advance), adding 100 μL of 10 μCi radioligand (normal saline solution), after vortexing the mixed solution for 5 minutes, centrifuging the solution on a centrifuge for 5 min (7000 r/min), after the centrifugation, disposing 100 μL of an organic phase and an aqueous phase in a PE test tube respectively, to measure the radioactivity count, and taking 100 μL of the organic phase from each group to be put in a 10 mL centrifuge tube, adding 900 μL of n-octanol and 1000 μL of PBS, performing vortex centrifugation as the above, to measure the radioactivity count, and repeating the operations 3 times in this way, to calculate an average value. log P=log (organic phase count/aqueous phase count)

Through measurement, it was obtained that the lipid water partition coefficient logP value of the radioligand [$^{18}$F] II-**15** was 1.64±0.12, which value accorded with the lipid solubility range for the drug to cross the blood brain barrier (BBB) to enter the brain.

Example 16: *In vitro* stability experiment of [$^{18}$F] II-15

The *in vitro* stability of the radioligand is of great importance for its further *in vivo* studies. Generally, the *in vitro* stability study is carried out in normal saline and animal serum. A specific method was as follows: respectively incubating 10 μCi of HPLC-purified radioligand [$^{18}$F] II-**15** and 100 μL of fetal bovine serum at 37 °C for 1 h and 2 h, adding 200 μL of acetonitrile thereto after finishing the incubation to fully precipitate proteins, then centrifuging at 40 °C for 5 min (7000 rpm), collecting supernatant, filtering the supernatant with filter membrane, and then performing HPLC analysis on 100 μL of the filtered supernatant; incubating 10 μCi of HPLC-purified radioligand [$^{18}$F] II-**15** with 100 μL of normal saline at room temperature for 1 h and 2 h, respectively, and then directly analyzing by HPLC.

Results of the *in vitro* stability experiment of radioligand [$^{18}$F] II-**15** are as shown in FIG. 6. It can be seen from FIG. 6 that [$^{18}$F] II-**15** shows very good stability in both normal saline and fetal calf serum. After 1 h (A in FIG. 6) and 2 h (B in FIG. 6) of the incubation in fetal calf serum at 37 °C, the radiochemical purities thereof were both greater than 98%; after 1 h (C in FIG. 6) and 2h (D in FIG. 6) of the incubation in normal saline at room temperature, the radiochemical purities thereof both remained greater than 98%.

Example 17: *In vivo* distribution experiment of [$^{18}$F] II-15 in animals

HPLC-purified [$^{18}$F] II-15 (10 μCi, dissolved in 0.1 mL of normal saline, containing 5% DMSO) was injected into normal Kunming mice (18-22 g, females, n=5) in a manner of tail vein injection, the mice were sacrificed by decapitation at the end of 5 min, 15 min, 30 min, 60 min and 90 min respectively, and dissected out to obtain blood, brain, heart, lung, liver, spleen, kidney, muscle, bone and tail, and wet weights of each organ were weighed and counted using a γ-counter. Uptake of each tissue was finally expressed as % ID/g, % ID/g=ID/g÷1%, where ID/g=radioactivity counts of tissue (counts)÷tissue mass (mg), 1%=average of 1% ID per time phase-tail radioactivity counts/100, and the *in vivo* distribution results of this radioligand are as shown in Table 11.

Table 11 *In Vivo* Distribution of Compound [$^{18}$F] II-15 in Female Kunming Mice (18-22 g)

| Organ | Absorption Amount (%ID/g wet weight) | | | | |
|---|---|---|---|---|---|
| | 5 min | 15 min | 30 min | 60 min | 90 min |
| blood | 1.43±0.06 | 1.24±0.16 | 1.03±0.07 | 0.68±0.01 | 0.52±0.06 |
| brain | 8.98±0.41 | 11.60±0.14 | 9.86±0.05 | 5.46±0.27 | 3.63±0.25 |
| heart | 8.37±0.14 | 4.45±0.62 | 3.56±0.31 | 2.19±0.16 | 1.71±0.13 |
| liver | 8.19±1.19 | 12.28±1.96 | 13.74±1.36 | 9.74±0.07 | 7.46±0.19 |
| spleen | 7.72±0.65 | 11.58±0.92 | 8.67±0.48 | 4.08±0.38 | 2.97±0.25 |
| lung | 70.52±7.86 | 20.23±1.51 | 18.30±1.56 | 7.64±0.99 | 4.99±0.45 |
| kidney | 15.92±0.24 | 11.54±0.79 | 8.06±0.69 | 4.85±0.443 | 4.26±0.19 |
| muscle | 5.51±0.65 | 3.48±0.40 | 2.36±0.30 | 1.79±0.06 | 1.32±0.11 |
| bone | 3.02±0.11 | 4.55±0.68 | 4.84±0.18 | 7.27±1.31 | 7.65±0.52 |
| brain/blood | 6.23 | 9.35 | 9.57 | 8.03 | 6.98 |

The data in the table are mean of five measurements ± standard deviation;

It can be seen from Table 11 that $^{18}$F-labeled radioligand [$^{18}$F] II-15 had very high initial brain uptake in the mouse brain, reaching an uptake value of 8.98±0.41% ID/g 5 min after the injection, showing the highest brain uptake value of 11.60±0.14% ID/g after 15 min; meanwhile, the radioligand showed a proper brain clearance rate, and 60 min and 90 min after administration, the uptake values in the brain were respectively reduced to 5.46±0.27% ID/g and 3.63±0.25% ID/g, which indicates that the compound has proper intracerebral kinetic properties; this result has shown a significant advantage over the [$^{18}$F] ASEM (the highest brain uptake value appears 5 min after administration, being 7.5% ID/g) that has been reported to enter clinical trials. In addition, [$^{18}$F] II-15 had a very low uptake value in blood, showing a very high brain/blood ratio, being 9.35 and 9.57 at 5 min and 15 min, respectively.

The *in vivo* defluorination phenomenon of the radioligand is a problem that has to be considered in the process of studying the F-18 labeled imaging agent. It can be seen from the experimental results in the above table that the bone absorption value of [$^{18}$F] II-15 was increased in the time range of study, but the increasing rate and the increasing amplitude were not very large, which indicates that the *in vivo* defluorination phenomenon of this compound was relatively weaker, and this phenomenon is not expected to interfere too much with the *in vivo* imaging studies of radioligands. In addition, there is also a high radioactivity uptake in other tissues and organs, for example, in the kidney and lung, the initial uptake value of the radioligands is quite high, but is gradually reduced over time and the clearance rate is very fast. This radioligand has better brain uptake properties than [$^{18}$F] ASEM.

Example 18: Experiment of brain region distribution of [$^{18}$F] II-15

HPLC-purified [$^{18}$F] II-15 (10 μCi, dissolved in 0.1 mL of normal saline, containing 5% DMSO) was injected into normal Kunming mice (28-32 g, females, n=5) in a manner of tail vein injection, the mice were sacrificed by dislocation of cervical vertebrae 5 min, 15 min, 30 min, 60 min, 90 min after the administration, respectively, the brains were rapidly dissected out and placed on ice, to remove blood streaks with ice-cold normal saline, then the cortex, corpus striatum, hippocampus, superior and inferior colliculus, thalamus, cerebellum and brain residual were dissected out regionally, the wet weight of each brain region was weighed and its radioactivity count was measured with a γ-counter. The radioligand uptake of each region is finally expressed by % ID/g, % ID/g=ID/g÷1%, where ID/g=radioactivity count of tissue (counts)÷tissue mass (mg), 1%=mean of 1% ID per time phase. The brain region distribution of this radioligand is shown in Table 12.

It can be seen from Table 12 that after 10 μCi [$^{18}$F] II-15 was injected into bodies of the mice, the radioligand showed higher uptake at cortex, corpus striatum and hippocampus where α7 nAChRs were the most abundant, and peaked at 9.39±0.24% ID/g, 8.37±0.27% ID/g and 6.31±0.82% ID/g, respectively, 30 min after administration, and the uptake values in these regions gradually declined over the following observation period; regions with moderate uptake were the superior and inferior colliculus and thalamus, and a region with the lowest uptake was the cerebellum (region where α7 nAChR distribution in mouse brain was the least). The brain region distribution characteristics were similar to [$^{18}$F] ASEM and consistent with the *in vitro* and *in vivo* distribution of α7 nAChR reported in the literature, and the uptake value in the region where α7 nAChR was dense had certain advantages over [$^{18}$F] ASEM: [$^{18}$F] ASEM reached the highest uptake value 5 min after the administration, and the uptake values in cortex, corpus striatum and hippocampus are 7.2% ID/g, 6.0% ID/g and 5.0% ID/g, respectively. Tissue/cerebellum ratios increased gradually throughout the whole

experiment and reached 2.5 (cortex), 2.9 (corpus striatum) and 3.6 (hippocampus) 90 min after the administration (Table 13), indicating that the radioligand not only has relatively higher brain uptake, but also has good brain regioselectivity

Table 12 Distribution of Compound [$^{18}$F] II-15 in Brains of Female Kunming Mice (28-32 g)

| Organ | Absorption (%ID/g wet weight) | | | | |
|---|---|---|---|---|---|
| | 5 min | 15 min | 30 min | 60 min | 90 min |
| cortex | 7.24±0.33 | 8.31±0.22 | 9.39±0.24 | 6.14±0.34 | 3.77±0.95 |
| hippocampus | 5.77±0.17 | 6.12±0.40 | 6.31±0.82 | 6.34±0.72 | 5.54±0.64 |
| corpus striatum | 6.99±0.31 | 6.55±0.25 | 8.37±0.27 | 5.01±0.31 | 4.37±0.18 |
| superior and inferior colliculus | 6.32±0.67 | 6.53±0.58 | 5.93±0.52 | 3.90±0.36 | 2.85±0.10 |
| thalamus | 5.74±0.69 | 5.66±0.34 | 5.80±0.47 | 4,89±0.04 | 3.19±0.26 |
| cerebellum | 5.83±0.01 | 5.10±0.35 | 4.53±0.14 | 2.69±0.10 | 1.53±0.10 |
| brain residual | 6.03±0.23 | 5.58±0.50 | 5.25±0.80 | 4.21±0.22 | 2.32±0.26 |

Table 13 Tissue/cerebellum Ratios of [$^{18}$F] II-15 in Each Tissue in Mouse Brain at Different Time Points

| Tissue | Tissue/cerebellum Ratio | | | | |
|---|---|---|---|---|---|
| | 5 min | 15 min | 30 min | 60 min | 90 min |
| cortex | 1.24 | 1.63 | 2.07 | 2.29 | 2.47 |
| hippocampus | 0.99 | 1.20 | 1.39 | 2.36 | 3.63 |
| corpus striatum | 1.20 | 1.28 | 1.85 | 1.87 | 2.86 |
| superior and inferior colliculus | 1.08 | 1.28 | 1.31 | 1.45 | 1.86 |
| thalamus | 0.98 | 1.11 | 1.28 | 1.82 | 2.10 |

Example 19: Selective experiment of [$^{18}$F] II-15 in mouse brain

The selectivity of radioligand for α4β2 nAChR was explored by subcutaneously injecting 1 mg/kg of cytisine (0.1 mL, with an equal volume of normal saline and 1,2-propanediol as solvents) 5 min in advance, whereas the selectivity for the 5-hydroxytryptamine receptor was explored by subcutaneously injecting 2 mg/kg of ondanstron (0.1 mL, with saline:DMSO=5:1 (V:V) as solvents) 10 min in advance. In the experiment, two groups of mice (n=5, Kunming females, 28-30 g) were injected with 0.1 mL (60 μCi) of [$^{18}$F] II-15 in a manner of tail vein injection, the mice in the blank control group were injected with 0.1 mL of corresponding solvent in the same manner. The mice were sacrificed by cervical dislocation 60 min after the administration, the brains were rapidly dissected out and placed on ice, after the blood streaks were washed out with ice-cold normal saline, the cortex, corpus striatum, hippocampus, superior and inferior colliculus, thalamus, cerebellum and brain residual were dissected out regionally, to measure the mass and radioactivity count of each region, and the final uptake was expressed as % ID/g.

Cytisine was a selective agonist of α4β2 nAChR, while Ondanstron was a selective antagonist of 5-hydroxytryptamine receptor. As can be seen from FIG. 7, there was no significant difference in the uptake of radioligand [$^{18}$F] II-15 between the experimental groups and control group, indicating that [$^{18}$F] II-15 had little binding to the α4β2 nAChR or 5-hydroxytryptamine receptor, and the radioligand had good selectivity for the α7 nAChR

Example 20: Rat PET imaging of [$^{18}$F] II-15

The radioligand [$^{18}$F] II-15 (0.3 mL, 200 μCi) was injected into bodies of female CD-1 rats (180-200 g) in a manner of tail vein injection, then the rats were anesthetized with 3% isoflurane to be coma, and the rats were fixed in a prone posture on a micro-PET/CT imaging instrument (Super Argus PET 4R L/CT 180) for small animals, maintaining the rats in an anesthetic state by employing 1% isoflurane in the scanning imaging process. Image acquisition was carried out respectively 15 min, 30 min and 60 min after the administration, to observe the distribution condition of [$^{18}$F] II-15 in the brains of the rats.

FIG. 8 shows the coronal, sagittal, and axial micro-PET images of the brains of female CD-1 rats 15 min, 30 min and 60 min after injection of [$^{18}$F] II-15 respectively. It can be seen from FIG. 8 that [$^{18}$F] II-15 has higher uptake in the rat brains, the distribution thereof in the brain is substantially consistent with *in vivo* distribution experiment result in animals. The highest uptake is achieved at 15 min, and the concentration of the radioligand is gradually reduced over

time, meanwhile, the retention in the brain is more proper, and enrichment of a certain concentration can still be observed 60 min after the administration. According to the good imaging result above, the [$^{18}$F] II-15 is suitable for being used as an $\alpha$7 nAChR PET imaging agent.

## Claims

1. A compound, having any one of the following formulas:

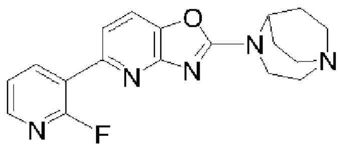,
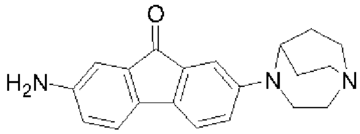,
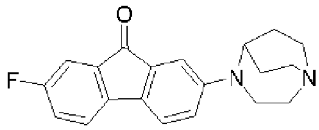,
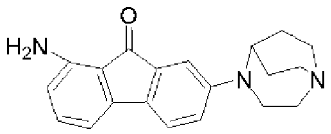,
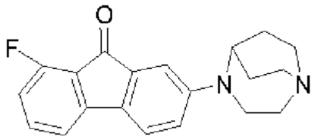, or
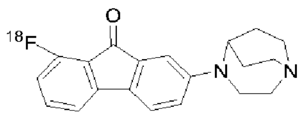.

2. The compound of claim 1, wherein the compound is used for preventing or treating a disease associated with $\alpha$7 nicotinic acetylcholine receptor, and the disease associated with $\alpha$7 nicotinic acetylcholine receptor is cognitive disorder being at least one selected from the group consisting of: presenile dementia, presenile Alzheimer's disease, senile dementia, dementia of the Alzheimer's type, Lewy body corpuscle dementia, micro-infarct dementia, AIDS-related dementia, HIV dementia, dementia associated with Lewy body, dementia associated with Down's syndrome, Pick's disease, mild cognitive dysfunction, age-related memory disorder, recent short-term memory disorder, age-related cognitive disorder, drug-related cognitive disorder, cognitive disorder associated with immunodeficiency syndrome, cognitive dysfunction associated with vascular diseases, schizophrenia, attention deficit disorder, attention deficit hyperactivity disorder and learning deficit disorder.

3. The compound of claim 1 for use in therapy.

4. A pharmaceutical composition containing a therapeutically effective amount of the compound of claim 1, and a pharmaceutically acceptable carrier.

5. The pharmaceutical composition of claim 4 for use in therapy.

6. Pharmaceutical composition for use in preventing or treating cognitive disorders, containing a therapeutically effective amount of the compound of claim 1, and a pharmaceutically acceptable carrier,
the cognitive disorders being at least one selected from the group consisting of: presenile dementia, presenile Alzheimer's disease, senile dementia, dementia of the Alzheimer's type, Lewy body corpuscle dementia, micro-infarct dementia, AIDS-related dementia, HIV dementia, dementia associated with Lewy body, dementia associated with Down's syndrome, Pick's disease, mild cognitive dysfunction, age-related memory disorder, recent short-term memory disorder, age-related cognitive disorder, drug-related cognitive disorder, cognitive disorder associated with immunodeficiency syndrome, cognitive dysfunction associated with vascular diseases, schizophrenia, attention deficit disorder, attention deficit hyperactivity disorder and learning deficit disorder.

7. Use of a compound as a PET imaging agent, wherein the compound has a structural formula of

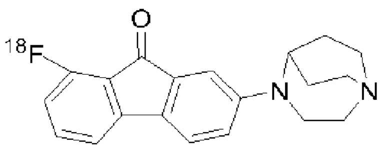.

**Patentansprüche**

1. Verbindung mit einer beliebigen der folgenden Formeln:

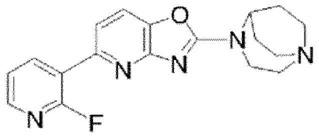, 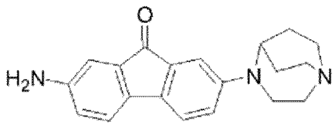, 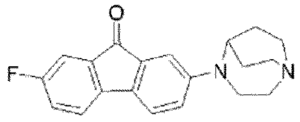,

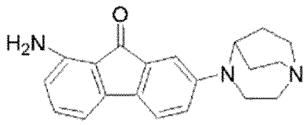, 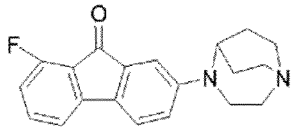, oder 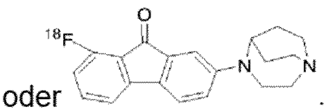.

2. Verbindung nach Anspruch 1, wobei die Verbindung zur Prävention oder Behandlung einer Krankheit, die mit $\alpha$7-Nikotinsäureacetylcholin-Rezeptor assoziiert ist, verwendet wird, wobei die Krankheit, die mit $\alpha$7-Nikotinsäureacetylcholin-Rezeptor assoziiert ist, eine kognitive Störung ist, die mindestens eine ist, die ausgewählt ist aus der Gruppe bestehend aus: präseniler Demenz, präseniler Alzheimer-Krankheit, seniler Demenz, Demenz vom Alzheimer-Typ, Lewy-Körperchen-Demenz, Mikroinfarkt-Demenz, AIDS-assoziierter Demenz, HIV-Demenz, mit Lewy-Körperchen assoziierter Demenz, mit Down-Syndrom assoziierter Demenz, Pick-Krankheit, leichter kognitiver Dysfunktion, altersbedingter Gedächtnisstörung, neu entwickelter Kurzzeitgedächtnisstörung, altersbedingter kognitiver Störung, drogenbedingter kognitiver Störung, mit Immundefizienzsyndrom assoziierter kognitiver Störung, mit Gefäßkrankheiten assoziierter kognitiver Dysfunktion, Schizophrenie, Aufmerksamkeitsdefizitsyndrom, Aufmerksamkeitsdefizit-Hyperaktivitätsstörung und Lernstörung.

3. Verbindung nach Anspruch 1 zur Verwendung in einer Therapie.

4. Pharmazeutische Zusammensetzung, die eine therapeutisch wirksame Menge der Verbindung nach Anspruch 1 und einen pharmazeutisch unbedenklichen Trägerstoff enthält.

5. Pharmazeutische Zusammensetzung nach Anspruch 4 zur Verwendung in einer Therapie.

6. Pharmazeutische Zusammensetzung zur Verwendung bei der Prävention oder Behandlung kognitiver Störungen, die eine therapeutisch wirksame Menge der Verbindung nach Anspruch 1 und einen pharmazeutisch unbedenklichen Trägerstoff enthält,
wobei es sich bei den kognitiven Störungen um mindestens eine handelt, die ausgewählt ist aus der Gruppe bestehend aus: präseniler Demenz, präseniler Alzheimer-Krankheit, seniler Demenz, Demenz vom Alzheimer-Typ, Lewy-Körperchen-Demenz, Mikroinfarkt-Demenz, AIDS-assoziierter Demenz, HIV-Demenz, mit Lewy-Körperchen assoziierter Demenz, mit Down-Syndrom assoziierter Demenz, Pick-Krankheit, leichter kognitiver Dysfunktion, altersbedingter Gedächtnisstörung, neu entwickelter Kurzzeitgedächtnisstörung, altersbedingter kognitiver Störung, drogenbedingter kognitiver Störung, mit Immundefizienzsyndrom assoziierter kognitiver Störung, mit Gefäßkrankheiten assoziierter kognitiver Dysfunktion, Schizophrenie, Aufmerksamkeitsdefizitsyndrom, Aufmerksamkeitsdefizit-Hyperaktivitätsstörung und Lernstörung.

7. Verwendung einer Verbindung als ein PET-Bildgebungsmittel, wobei die Verbindung eine Strukturformel von

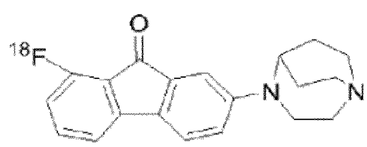

aufweist.

## Revendications

1. Composé, répondant à l'une des formules suivantes :

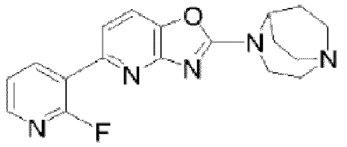
,
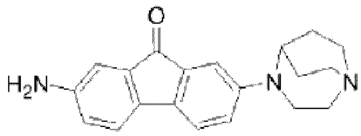
,
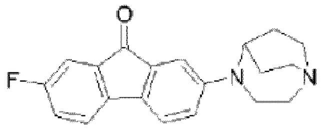
,
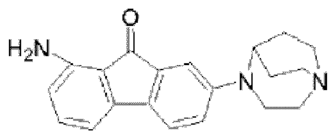
,
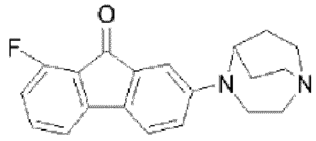
, ou
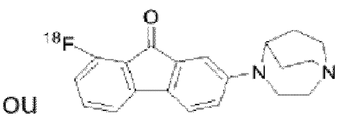
.

2. Composé selon la revendication 1, dans lequel le composé est utilisé pour prévenir ou traiter une maladie associée au récepteur nicotinique de l'acétylcholine a7, et la maladie associée au récepteur nicotinique de l'acétylcholine $\alpha$7 est un trouble cognitif étant au moins un trouble choisi dans le groupe constitué de : la démence présénile, la maladie d'Alzheimer présénile, la démence sénile, la démence de type Alzheimer, la démence à corpuscules de corps de Lewy, la démence liée à des micro-infarctus, la démence liée au SIDA, la démence liée au VIH, la démence associée aux corps de Lewy, la démence associée au syndrome de Down, la maladie de Pick, un dysfonctionnement cognitif léger, un trouble de la mémoire lié à l'âge, un trouble récent de la mémoire immédiate, un trouble cognitif lié à l'âge, un trouble cognitif lié aux médicaments, un trouble cognitif associé au syndrome d'immunodéficience, un dysfonctionnement cognitif associé aux maladies vasculaires, la schizophrénie, le trouble du déficit de l'attention, le trouble d'hyperactivité avec déficit de l'attention et un trouble de déficit d'apprentissage.

3. Composé selon la revendication 1 pour une utilisation en thérapie.

4. Composition pharmaceutique contenant une quantité thérapeutiquement efficace du composé selon la revendication 1, et un support pharmaceutiquement acceptable.

5. Composition pharmaceutique selon la revendication 4 pour une utilisation en thérapie.

6. Composition pharmaceutique pour une utilisation dans la prévention ou le traitement de troubles cognitifs, contenant une quantité thérapeutiquement efficace du composé selon la revendication 1, et un support pharmaceutiquement acceptable,
les troubles cognitifs étant au moins un trouble choisi dans le groupe constitué de : la démence présénile, la maladie d'Alzheimer présénile, la démence sénile, la démence de type Alzheimer, la démence à corpuscules de corps de Lewy, la démence liée à des micro-infarctus, la démence liée au SIDA, la démence liée au VIH, la démence associée aux corps de Lewy, la démence associée au syndrome de Down, la maladie de Pick, un dysfonctionnement cognitif léger, un trouble de la mémoire lié à l'âge, un trouble récent de la mémoire immédiate, un trouble cognitif lié à l'âge, un trouble cognitif lié aux médicaments, un trouble cognitif associé au syndrome d'immunodéficience, un dysfonctionnement cognitif associé aux maladies vasculaires, la schizophrénie, le trouble du déficit de l'attention, le trouble d'hyperactivité avec déficit de l'attention et un trouble de déficit d'apprentissage.

7. Utilisation d'un composé comme agent d'imagerie par TEP, dans lequel le composé a une formule développée de

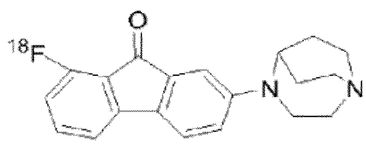
.

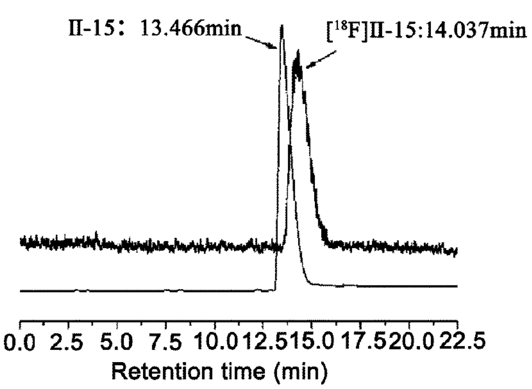


FIG.1

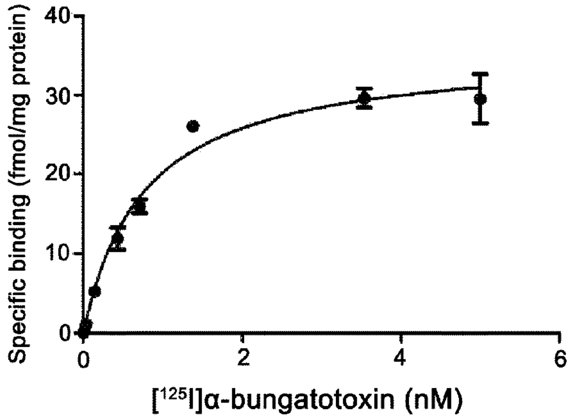


FIG.2

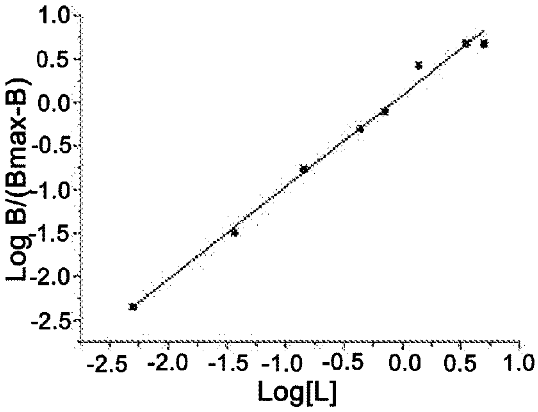


FIG.3

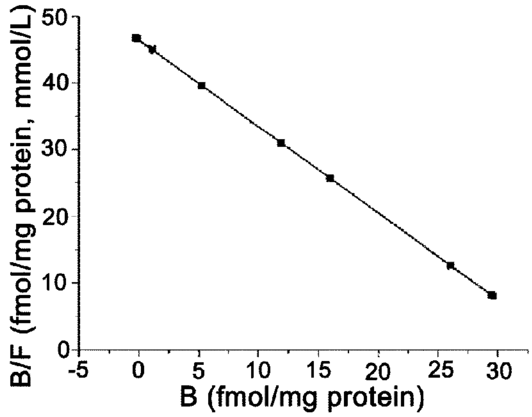


FIG.4

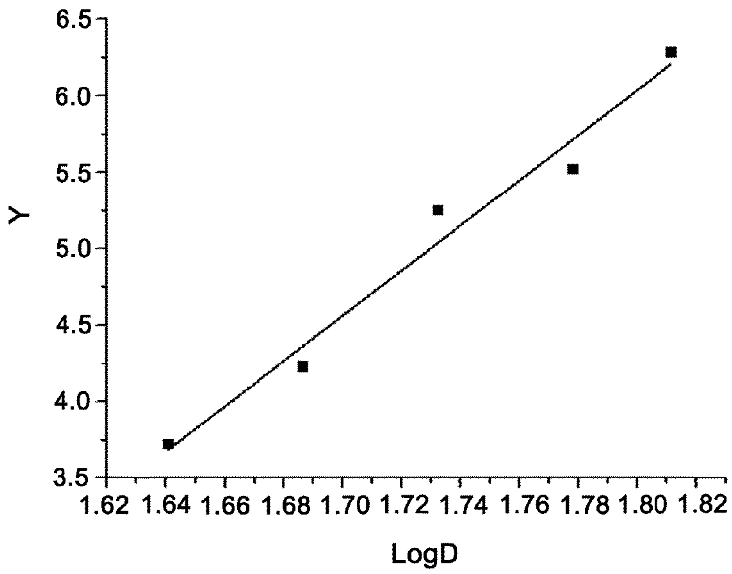


FIG.5

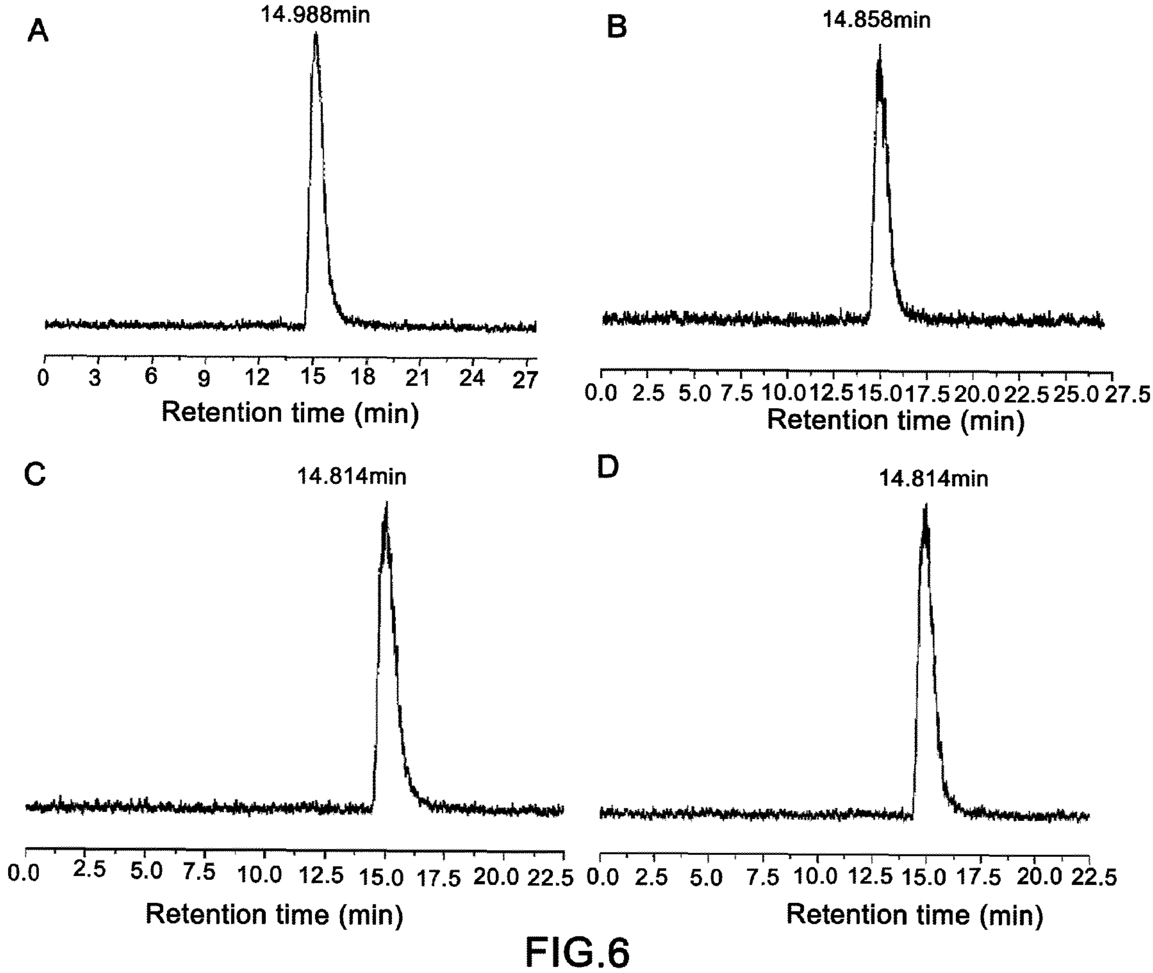
A
14.988min
0 3 6 9 12 15 18 21 24 27
Retention time (min)
B
14.858min
0.0 2.5 5.0 7.5 10.0 12.5 15.0 17.5 20.0 22.5 25.0 27.5
Retention time (min)
C
14.814min
0.0 2.5 5.0 7.5 10.0 12.5 15.0 17.5 20.0 22.5
Retention time (min)
D
14.814min
0.0 2.5 5.0 7.5 10.0 12.5 15.0 17.5 20.0 22.5
Retention time (min)

FIG.6

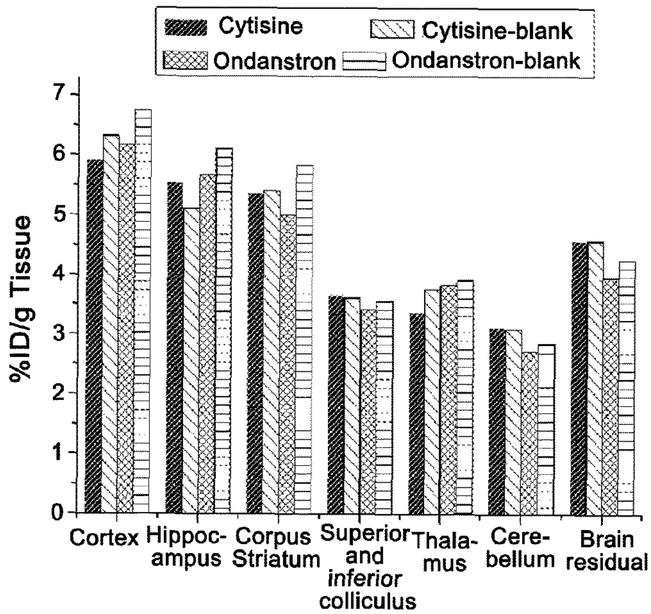
Cytisine
Cytisine-blank
Ondanstron
Ondanstron-blank
%ID/g Tissue
0 1 2 3 4 5 6 7
Cortex
Hippocampus
Corpus Striatum
Superior and inferior colliculus
Thalamus
Cerebellum
Brain residual

FIG.7

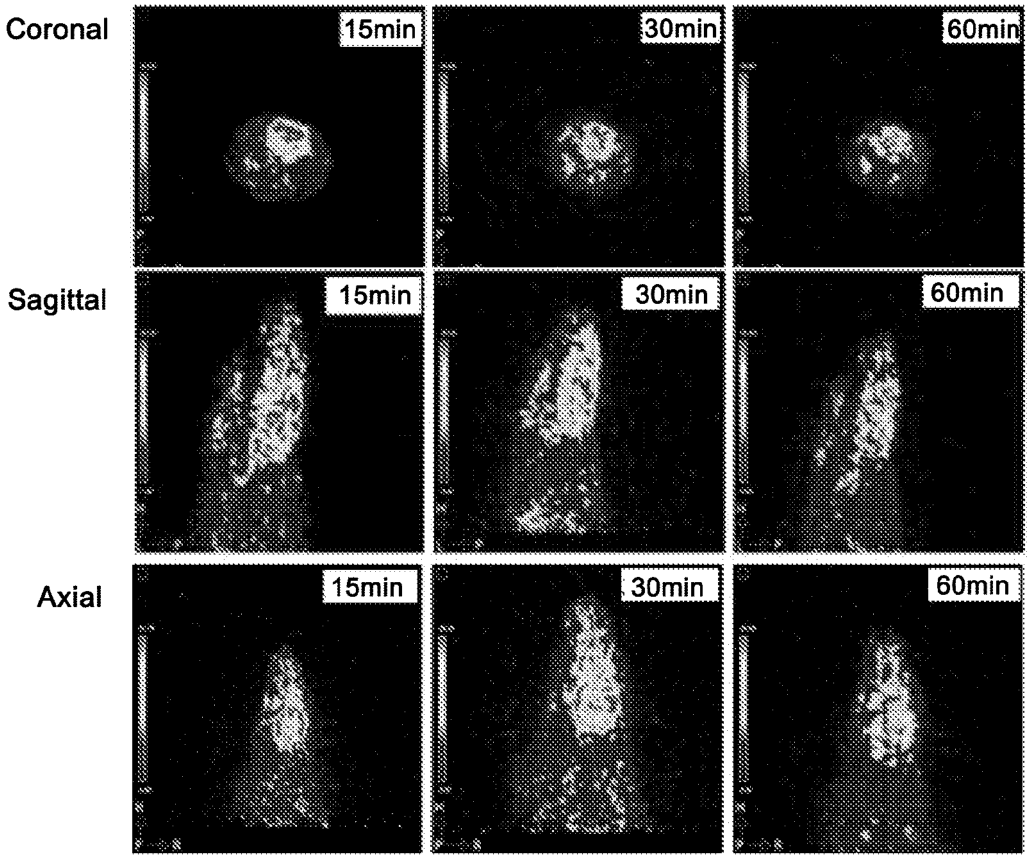
Coronal
15min
30min
60min
Sagittal
15min
30min
60min
Axial
15min
30min
60min

FIG.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201710395513